(19)

**Europäisches Patentamt**
**European Patent Office**
**Office européen des brevets**

(11) **EP 4 353 179 A1**

(12) **EUROPEAN PATENT APPLICATION**
published in accordance with Art. 153(4) EPC

(43) Date of publication:
**17.04.2024 Bulletin 2024/16**

(21) Application number: **22819182.1**

(22) Date of filing: **02.04.2022**

(51) International Patent Classification (IPC):
**A61B 34/30** (2016.01)

(52) Cooperative Patent Classification (CPC):
**A61B 34/30; A61B 34/35; A61B 34/37**

(86) International application number:
**PCT/CN2022/085047**

(87) International publication number:
**WO 2022/257577 (15.12.2022 Gazette 2022/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.06.2021 CN 202110628500**

(71) Applicant: **Shenzhen Edge Medical Co., Ltd.**
**Shenzhen, Guangdong 518000 (CN)**

(72) Inventors:
• **WANG, Shenhui**
  **Shenzhen, Guangdong 518000 (CN)**
• **YE, Guoqiang**
  **Shenzhen, Guangdong 518000 (CN)**

(74) Representative: **Biallo, Dario et al**
**Barzanò & Zanardo Milano S.p.A.**
**Via Borgonuovo, 10**
**20121 Milano (IT)**

(54) **SURGICAL ROBOT AND CONTROL METHOD THEREFOR**

(57) A surgical robot and a control method. The surgical robot comprises: at least one arm provided with a joint assembly and a driving mechanism that drives the joint assembly to move and comprising a first arm; and at least one processor (501) electrically connected to the driving mechanism and configured to: in response to the external force received by the first arm, adjust an output torque of the driving mechanism corresponding to a first joint assembly in the joint assembly of the first arm to balance a gravitational torque of a load at the distal end of the first joint assembly, and restore the joint position of the first joint assembly or brake the first joint assembly. The first arm can move more flexibly under the action of the external force so as to reduce the impact of the external force, resetting or braking can be achieved after the external force disappears, and the reliability can be improved.

FIG. 6

EP 4 353 179 A1

**Description**

The present disclosure claims the benefit of Chinese Patent Application NO. 202110628500.X, entitled "surgical robot, and anti-collision method for arms thereof and anti-collision system for arms thereof', filed with China National Intellectual Property Administration on June 7, 2021, and the entire contents of which are incorporated herein by reference.

TECHNICAL FIELD

The present disclosure relates to medical instruments, more particularly to a surgical robot and a control method therefor.

BACKGROUND

Minimally invasive surgery refers to a surgical method for performing surgery in a human body cavity using modern medical instruments such as laparoscopes, thoracoscopes, and related devices. Compared with traditional surgery modes, minimally invasive surgery has advantages of resulting in small trauma, light pain, fast recovery, and the like.

With advances in science and technology, minimally invasive surgical systems are increasingly mature and widely used. A surgical system generally includes a master control console and a slave operating device, the master control console is configured for controlling the slave operating device by sending control instructions to the slave operating device according to the doctor's manipulation. The slave operating device is configured for executing corresponding surgical operation responding to the control commands sent by the master control console.

A surgical robot generally includes a number of arms, in use or not in use, the arms generally maintain a high degree of stiffness. When some of the arms are applied with external force such as collisions, due to the collisions, the structures of the arms which maintain a high degree of stiffness may be easily damaged, and the reliability of the surgical procedures may be easily affected.

SUMMARY

In view of the above, the present disclosure provides a surgical robot and a control method therefor, which can make the arms of the surgical robot move sensitively under the effect of the external force to mitigate the impact of the external force, and be restored or braked without the external force, and can improve the reliability.

The present disclosure provides surgical robot, which includes: at least one arm and at least one processor. The at least one arm includes a joint assembly and an actuating device for actuating the joint assembly, the at least one arm includes a first arm. The at least one processor is electrically connected to the actuating device, and the at least one processor is configured for: adjusting, in response to an external force received by the first arm, an output torque of the actuating device corresponding to a first joint assembly of the joint assembly of the first arm to balance a gravitational torque of a load of a distal end of the first joint assembly, and to restore a joint position of the first joint assembly or brake the first joint assembly.

The present disclosure also provides a control method for a surgical robot, the surgical robot includes at least one arm, the at least one arm includes a joint assembly and an actuating device for actuating the joint assembly, the at least one arm includes a first arm; and the control method includes steps of: adjusting, in response to an external force received by the first arm, an output torque of the actuating device corresponding to a first joint assembly of the j oint assembly of the first arm to balance a gravitational torque of a load of a distal end of the first joint assembly, and to restore a joint position of the first joint assembly or brake the first joint assembly.

The present disclosure also provides a computer-readable storage medium, suitable for a surgical robot, the surgical robot includes at least one arm, the at least one arm includes a joint assembly and an actuating device for actuating the joint assembly, and the computer-readable storage medium stores a computer program, which is configured for being loaded and executed by a processer to implement the steps of the control method according to any one of the above embodiments.

The present disclosure also provides a control system of a surgical robot, suitable for a surgical robot, the surgical robot includes at least one arm, the at least one arm includes a joint assembly and an actuating device for actuating the joint assembly, it also includes a memory, configured for storing a computer program; and a processer, configured for loading and executing the computer program; in which the computer program is configured for being loaded and executed by the processor to implement the steps of the control method according to any one of the above embodiments.

The present disclosure has the following beneficial effects:

In response to that the first arm of the surgical robot receives the external force, by adjusting the output torque of the actuating device corresponding to the first joint assembly of the joint assembly to balance the gravitational torque of the load of the distal end of the first joint assembly, and to restore the joint position of the first joint assembly or brake the first joint assembly, it can make the first arm move sensitively under the effect of the external force to mitigate the impact of the external force, and be restored or braked without the external force, and can improve the reliability

BRIEF DESCRIPTION OF THE DRAWINGS

FIG. 1 is a schematic structure diagram of a slave operating device of a surgical robot according to an embodiment of the present disclosure;
FIG. 2 is a schematic structure diagram of a master control console of the surgical robot according to an embodiment of the present disclosure;
FIG. 3 is a schematic structure diagram of the master control console of the surgical robot according to another embodiment of the present disclosure;
FIG. 4 is a schematic diagram of a master wrist of an operating device of the master control console illustrated in FIG. 3 according to an embodiment of the present disclosure;
FIG. 5 is a schematic structure diagram of an effective space of a first arm of the surgical robot according to an embodiment of the present disclosure;
FIG. 6 to FIG. 20 are respectively flow charts of an anti-collision method for the surgical robot according to different embodiments of the present disclosure; and
FIG. 21 is a schematic structure diagram of a control apparatus of the surgical robot according to an embodiment of the present disclosure.

DETAILED DESCRIPTION OF THE EMBODIMENTS

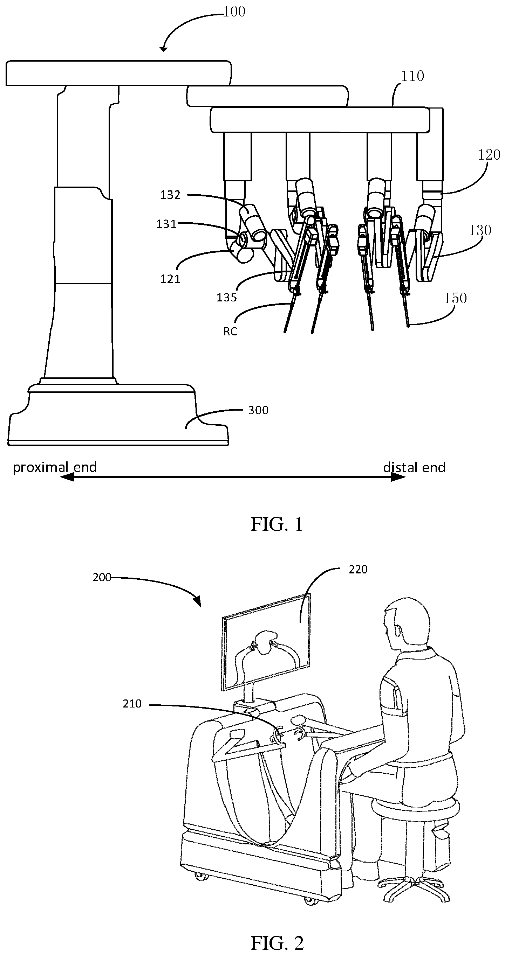

In order to facilitate the understanding of the present disclosure, the present disclosure will be more fully described below with reference to the relevant accompanying drawings. Preferable embodiments of the present disclosure are given in the accompanying drawings. However, the present disclosure may be implemented in many different manners and is not limited to the embodiments described herein. Rather, these embodiments are provided for the purpose of providing a more thorough and comprehensive understanding of the disclosed contents of the present disclosure.
It should be noted that when an element is referred to as being "disposed on" another element, it may be directly on the other element or intervening elements may also be present. When an element is considered to be "connected" to another element, it may be directly connected to another element or intervening elements may be present at the same time. When an element is considered to be "coupled" to another element, it may be directly coupled to another element or intervening elements may be present at the same time. As used herein, the terms "vertical", "horizontal", "left", "right" and the like are intended for purposes of illustration only and are not intended to be limiting. As used herein, the terms "distal end" and "proximal end" are common terms in the art of interventional medical devices, where "distal end" refers to the end away from the operator during the surgical procedure, and the "proximal end" refers to the end close to the operator during the surgical procedure. The term "first/second" and the like as used in this disclosure indicates a component and a group of two or more components having common characteristics.
Unless defined otherwise, all technical and scientific terms used herein have the same meaning as commonly understood by one of ordinary skill in the art. The terminology used herein is for the purpose of describing particular embodiments only and is not intended to be limiting of the disclosure. As used herein, the term "and/or" includes any and all combinations of one or more of the relevant listed items. As used in this disclosure, the term "each" includes one or more than two. As used in this disclosure, the term "a plurality of" includes more than two.
Refer to FIG. 1 to FIG. 2, which are respectively the schematic structure diagrams of a slave operating device and a master control console of a surgical robot according to an embodiment of the present disclosure.
The surgical robot includes a slave operating device 100 and a master control console 200 which is configured for controlling the slave operating device 100. The master control console 200 includes an operating device 210 and a display device 220, and a doctor sends control commands to the slave operating device 100 by operating the operating device 210, so that the slave operating device 100 performs corresponding operations according to the control commands sent by the doctor by operating the operating device 210, and the doctor observes a surgical area through the display device 220. The operating device 210 generally may be a motion input device, for example, it may be at least one of a linkage type mechanical handle, a magnetic navigation handle, and a gesture recognition device (such as soft kinetic of Sony).

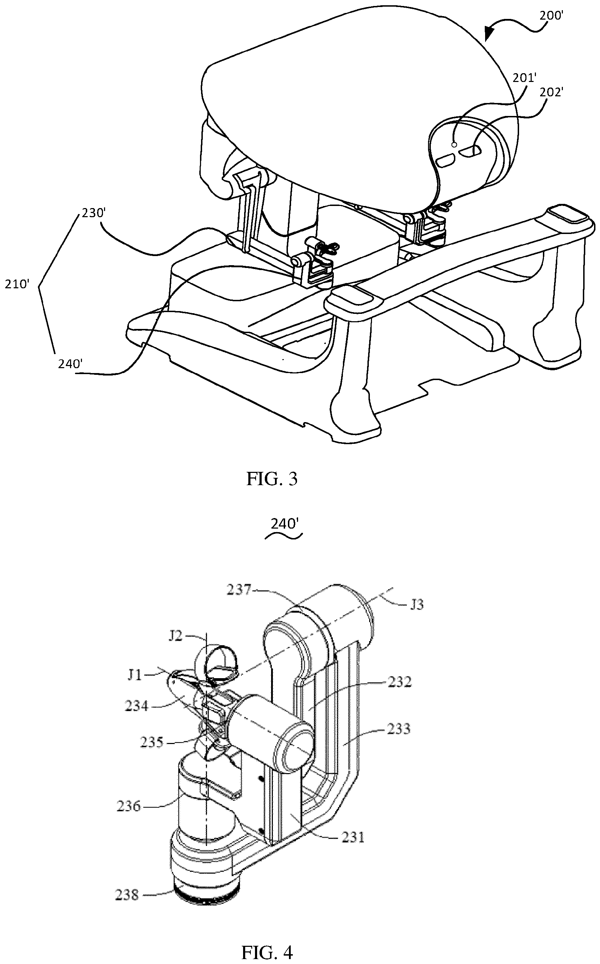

FIG. 3 illustrates the structure diagram of the master control console of the surgical robot according to another embodiment. The master control console 200' includes an operating device 210' and a display (not shown), the doctor may observe the operating image displayed on the display while manipulating the operating device 210', and in turn manipulating (i.e. controlling) the slave operating device 100 to move. The operating device 210' includes an arm, the arm includes a joint assembly and an actuating device which actuates the joint assembly to move, the actuating device and the joint assembly may be actuated by means of a filament structure. In this case, the filament structure may include a filiform structure or a ribbon structure. For example, the filament structure may include a steel wire or a steel strip. In this case, the actuating device includes, for example, a motor.

The surgical robot also includes at least one processor, the at least one processor is electrically connected to the master control console 200 and/or the master control console 200', and also the at least one processor is electrically connected to the slave operating device 100. For example, the at least one processor is electrically connected to the operating device 210, the display device 220 and the slave operating device 100. For example, the at least one processor is electrically connected to the operating device 210' and the slave operating device 100, in this case, the at least one processor is electrically connected to the actuating devices of the operating device 210'. The processors may be integrated in the master control console 200, may also be integrated in the slave operating device 100, may also be distributed in the master control console 200 and the slave operating device 100, and may also be deployed in the cloud. The master control console 200 (200') and the slave operating device 100 may be deployed in the same room or different rooms.

The operating device 200' may include at least one arm. The slave operating device 100 may include at least one arm, for ease of description, the at least one arm can be divided into different parts to be named separately according to the functions and/or the modular division. Each of the parts may be named as a sub-arm, each of the sub-arms includes at least one joint assembly which includes a linkage and a joint, and the joint assembly may be a prismatic joint assembly and/or a rotary joint assembly. The position and/or the orientation of the distal end of the corresponding sub-arm can be adjusted by adjusting the joint assembly. The sub-arms may be configured to be adjusted independently or coordinately, depending on demands.

Continue to FIG.3, for example, the arm of the operating device 200' may be divided into two parts, each of the parts corresponds to one sub-arm. For example, the two sub-arms of the operating device 200' include a master arm 230' and a master wrist 240' which connects to the master arm 230', each of the master arm 230' and the master wrist 240' respectively includes a joint assembly and an actuating device which actuates the joint assembly, and the joint assembly and the actuating device are actuated by means of a filament structure. In some embodiments, the movement of the joint assembly of the master arm 230' mainly affects the position of the distal end of the master wrist 240', the movement of the joint assembly of the master wrist 240' mainly affects the orientation (that is, the posture) of the distal end of the master wrist 240'.

The master control console 200' may also include multiple types of sensors, these sensors are respectively coupled to the at least one processor. In these sensors, sensors for detecting (that is, sensing) the joint position of the joint assemblies of the operating device 210' are included, the sensor for the use may be, for example, the position sensor for detecting the joint position of the joint assemblies of the operating device 210', and may also be at least one of the encoder and the position sensor for detecting the position of the actuating devices which actuate the joint assemblies of the operating device 210'. And/or, in these sensors, are included sensors for detecting the current signals of the actuating devices of the operating device 210' for actuating the joint assemblies to move, the sensor for the use may be, for example, at least one of the current sensor and the voltage sensor. And/or, in these sensors, are included sensors for detecting the force signal of the joint assemblies of the operating device 210', the sensor for the use may be, for example, at least one of the three-dimensional force sensor, the six-dimensional force sensor, and a combination of multiple of the one-dimensional force sensors. And/or, in these sensors, is included the proximity sensor 201' for detecting whether the doctor is close to the master control console 200', for example, the proximity sensor 201' may use the infrared sensor, the proximity sensor 201' may be arranged nearby the observing window 202' of the master control console 200', and in response to that the doctor's head is close to the observing window 202', the head will be detected by the proximity sensor 201'.

FIG. 4 is a schematic diagram of the master wrist of the operating device of the master control console illustrated in FIG. 3, in which, the master wrist 240' includes a plurality of degrees of freedom (DOFs), generally, it includes at least three DOFs. The master wrist 240' includes a first linkage 231, a second linkage 232, a third linkage 233, a fixture 234, a first rotary joint 235, a second rotary joint 236, a third rotary joint 237, and a fourth rotary joint 238. The fixture 234 is mounted to one end of the first linkage 231 through the first rotary joint 235. The other end of the first linkage 231 is mounted to one end of the second linkage 232 through the second rotary joint 236. The other end of the second linkage 232 is mounted to one end of the third linkage 233 through the third rotary joint 237. The other end of the third linkage 233 is mounted to the master arm 230' through the fourth rotary joint 238.

The fixture 234 is rotationally connected to the first linkage 231 through the first rotary joint 235, so that the fixture 234 may revolve around the first rotation axis J1 of the first rotary joint 235. The first linkage 231 is rotationally connected to the second linkage 232 through the second rotary joint 236, so that the first linkage 231 may revolve around the second rotation axis J2 of the second rotary joint 236. The second linkage 232 is rotationally connected to the third linkage 233 through the third rotary joint 237, so that the second linkage 232 may revolve around the third rotation axis J3 of the third rotary joint 237. The third linkage 233 is rotationally connected to the master arm 230' through the fourth rotary joint 238, so that the third linkage 233 may revolve around the rotation axis of the fourth rotary joint 238.

The rotation axes of the first rotary joint 235, the second rotary joint 236 and the third rotary joint 237 corresponding to the first rotation axis J1, the second rotation axis J2 and the third rotation axis J3 are intersected at one point. The master wrist 240' uses the design of multiple axes intersecting at one point, so that the orientation and the position of the whole operating device 200' (that is, a master assembly) are relatively decoupled, for facilitating kinematic solutions. For example, it is possible to make the roll (that is, rotate) joint assembly to include the first rotary joint 235, to make the pitch joint assembly to include the second rotary joint 236, and to make the yaw joint assembly to include the third rotary joint 237.

For example, the first linkage 231, the second linkage 232, and the third linkage 233 may be L-shaped rods, each of the linkages includes two ends which are connected mutually vertically, and the two ends of the each linkage are respectively connected to different rotary joints.

The fixture 234 can perform movements in a plurality of DOFs through a plurality of the linkages (the first linkage 231, the second linkage 232, and the third linkage 233) and a plurality of the rotary joints (the first rotary joint 235, the second rotary joint 236, the third rotary joint 237, and the fourth rotary joint 238). In other embodiments, the number of the linkages and the rotary joints may be configured according to the actual requirement about the DOFs of the fixture 234.

Continue to FIG. 3, the master arm 230' includes a mounting end and a connection end, the mounting end may be fixedly connected to the support base of the master control console 100. The master arm 230' includes at least one DOF. The master wrist 240' may be actively connected to the connection end of the master arm 230' through the fourth rotary joint 238. The master wrist 240' allows the user to perform corresponding operations, such as rotating, clamping, or the like. The fixture 234 includes open/close-related DOF, when the motion of the open/close-related DOF of the fixture 234 is mapped to the surgical instrument, the open/close-related operation (such as clamping, cutting, or the like) of the end effector can be controlled. When the motion of rotating DOF of the fixture 234 by revolving around the first rotation axis J1 of the first rotary joint 235 is mapped to the surgical instrument, the rolling motion of the end effector of the surgical instrument can be controlled.

In some embodiments, the processor is configured for:

When the doctor is in an unreadiness state, in response to the external force received by the operating device, adjusting the output torque of the actuating device corresponding to the first joint assembly of the joint assembly of the operating device to balance the gravitational torque generated by the load of the distal end of the first joint assembly, and to restore the joint position of the first joint assembly or brake the first joint assembly.

In this case, the processor determines in real-time whether the doctor is in the unreadiness state, the doctor being in the unreadiness state indicates that the operating device is in the idle state, which indicates the doctor does not have the true intention to manipulate the operating device. For example, the doctor being in the unreadiness state may include at least one of the following types of conditions.

The doctor is not observing the display of the display device;

Generally, when manipulating the slave operating device by the operating device, the doctor needs to observe the display of the display device, so that it is possible to make a state where the doctor is not observing the display of the display device to be one of the conditions indicating that the doctor is in the unreadiness state.

Whether the doctor is observing the display of the display device can be determined by a plurality of methods. For example, such methods may include whether the head of the doctor is in proximity to the display, and/or, whether the eyes of the doctor are gazing at the display.

For example, a sensor 201' for detecting whether the head of the doctor is in proximity to the observing window 202', which is used for observing the display surface of the display can be configured at the edge area of the observing window 202' (including the observing window 202' itself) of the master control console 200', and the sensor 201' is electrically connected to the processor. The sensor 201' may include a proximity sensor such as an infrared ray sensor. In response to that the head of the doctor is not in proximity to the observing window 202', the sensor 201' would sense a first signal, and in response to that the head of the doctor is in proximity to the observing window 202', the sensor 201' would sense a second signal. In this way, whether the head of the doctor is in proximity to the observing window 202' can be determined according to the signal sensed by the sensor 201'. In this case, in response to that the sensor 201' senses the first signal, it can be determined that the head of the doctor is not in proximity to the observing window 202', and in response to that the sensor 201' senses the second signal, it can be determined that the head of the doctor is in proximity to the observing window 202'.

For another example, an eye-gaze tracking device for detecting whether the at least one eye of the doctor are gazing at the display can be configured at the edge area of the display surface (including the display surface itself) of the display device, and the eye-gaze tracking device is electrically connected to the processor. In response to that the eye-gaze tracking device tracks the eyes, it can be determined that the doctor is gazing at the display, and in response to that the eye-gaze tracking device fails to track the eyes, it can be determined that the doctor is not gazing at the display. The working principle of the eye-gaze tracking device may, for example, be as follows: the eye-gaze tracking device sends an infrared ray, and captures a reflected infrared ray from the eyes. It can be determined that the doctor is gazing at the display in response to that the eye-gaze tracking device captures the inflected infrared ray, and it can be determined that the doctor is not gazing at the display in response to that the eye-gaze tracking device fails to capture the inflected infrared ray.

The doctor fails to activate the operating device;

When the doctor manipulates the slave operating device by the operating device, activation of the operating device is generally required firstly, so that it is possible to make a state where the doctor fails to activate the operating device to be one of the conditions indicating that the doctor is in the unreadiness state.

Whether the doctor activates the operating device can be determined by a plurality of methods. For example, such methods may include whether the doctor pinches the fixture of the operating device, and/or, whether the doctor rotates the fixture of the operating device.

For example, in response to that the angle of opening and closing of the fixture of the operating device 210' reaches the first threshold, and/or, in response to that the times of opening and closing of the fixture of the operating device 210' reaches the second threshold, it can be determined that the doctor pinches the fixture of the operating device, and in response to that the angle of opening and closing of the fixture 234 fails to reach the first threshold, and/or, in response to that the times of opening and closing of the fixture 234 fails to reach the second threshold, it can be determined that the doctor does not pinch the fixture of the operating device. It can be determined that the doctor activates the operating device in response to that the doctor pinches the fixture of the operating device, and it can be determined that the doctor fails to activate the operating device in response to that the doctor does not pinch the fixture of the operating device.

For another example, in response to that the angle of rotation of the first rotary joint 235 of the operating device 210' reaches the first threshold, it can be determined that the doctor rotates the fixture of the operating device, and in response to that the angle of rotation of the first rotary joint 235 of the operating device 210' fails to reach the third threshold, it can be determined that the doctor fails to rotate the fixture of the operating device. It can be determined that the doctor activates the operating device in response to that the doctor rotates the fixture of the operating device, and it can be determined that the doctor fails to activate the operating device in response to that the doctor does not rotate the fixture of the operating device.

The doctor is not seated in place;

Generally, when manipulating the slave operating device by the operating device, the doctor is required to be seated in place, and/or, the doctor is seated within a reasonable range relative to the operating device and/or the display device, so that it is possible to make a state where the doctor is not seated in place to be one of the conditions indicating that the doctor is in the unreadiness state.

The surgical robot includes a seat for the doctor to seat.

For example, the seat is provided with a sensor for detecting whether the doctor is seated in place, and the sensor is electrically connected to the processor. For example, the sensor includes a pressure sensor. In response to that a pressure value sensed by the sensor reaches the first threshold, it can be determined that the doctor is seated in place, and in response to that the pressure value sensed by the sensor fails to reach the first threshold, it can be determined that the doctor is not seated in place.

For another example, the surgical robot includes a distance measuring device which is electrically connected to the processor, the distance measuring device is configured for detecting a distance of the seat and/or the doctor from the operating device and/or the display device, for example, the distance measuring device includes a laser radar or an infrared distance sensor. In response to that the distance of the seat and/or the doctor from the operating device and/or the display device sensed by the distance measuring device reaches the first threshold, it can be determined that the doctor is not seated in place within the reasonable range, and in response to that the distance of the seat and/or the doctor from the operating device and/or the display device sensed by the distance measuring device fails to reach the first threshold, it can be determined that the doctor is seated within the reasonable range.

The doctor fails to be certified by an user authentication system;

When manipulating the slave operating device by the operating device, the doctor is generally required to pass certification of a user authentication system of the surgical robot firstly, so that it is possible to make a state where the doctor fails to be certified by the user authentication system to be one of the conditions indicating that the doctor is in the unreadiness state.

For example, the state where the doctor fails to be certified by the user authentication system includes: that the user authentication system fails to obtain a login information (including an account and a password) of the doctor, and/or, that the user authentication system obtains the login information of the doctor, but the login information fails to match with a pre-recorded information of the doctor.

The situations under the above items (1) - (4) may be configured to be used separately as conditions indicating that the doctor is in the unreadiness state, and may also be configured to be used in any combinations with each other as conditions indicating that the doctor is in the unreadiness state.

In this case, in response to that the change of the joint position of the first rotary joint 235 reaches a first activation threshold, it can be determined that the master wrist 240' is activated by the doctor. In this case, the external force received by the operating device includes an external force applied by a person on the operating device, and/or, an external force (such as an external force caused by the collisions between objects) applied by an object on the operating device.

The "balance" described in the present disclosure, generally means a relatively balanced relationship, and allows the presence of a torque deviation. For a same first joint assembly, the balance may be considered to be realized when the following formula is satisfied:

$$ x = \left| F_1 - F_2 \right| \leq \Delta x_, $$

in which, $x$ is the torque deviation, F1 is the output torque of the actuating device, F2 is the gravitational torque of the load of the distal end of the corresponding first joint assembly, $\Delta x$ is a torque deviation threshold. For example, the range of value of $\Delta x$ may be [0, 10] N/m.

In this case, as long as $x \leq \Delta x$, the output torque of the actuating device can be considered to balance the gravitational torque of the load of the distal end of the corresponding joint assembly.

And then, when the operating device is collided, the adjusted joint assembly of the operating device, which is the first joint assembly, can be deflected and/or moved easily under the effect of the external force, to effectively buffer the external force by the changes in position and/or in orientation. More particularly, by adjusting the output torque of the actuating device corresponding to the first joint assembly to balance the gravitational torque of the load of the distal end of the first joint assembly, and to restore the joint position of the first joint assembly or brake the first joint assembly, not only can the operating device be released from a rigid state and become flexible, so as to protect the filiform structures from being pulled apart, but also the corresponding first joint assembly can be restored or braked after the forceful motion of the corresponding first joint assembly.

In some embodiments, the first joint assembly of the operating device may be formed by a joint assembly which is configured by the system or the user. In the embodiments, which joint assembly of the operating device is exactly included by the first joint assembly is pre-determined (that is, pre-configured) before the external force is received. For example, the first joint assembly of the operating device can be obtained from the system files, and the first joint assembly of the operating device may also be configured by the user configuration interface. In this case, the first joint assembly generally means a type of joint assembly, and the number of the first joint assembly includes at least one. For example, when the first joint assemblies include at least two joint assemblies, the processor can be configured to adjust the output torque of the actuating device corresponding to each first joint assembly to balance the gravitational torque of the load of the distal end of the corresponding first joint assembly respectively.

For example, the joint assembly of the master arm 230' and the joint assembly of the master wrist 240' can be configured as the first joint assemblies. When the first joint assemblies include a pitch joint assembly corresponding to a pitch DOF and a yaw joint assembly corresponding to a yaw DOF, assuming that the external force received by the operating device has component forces in the pitch DOF and in the yaw DOF, by adjusting the output torque of the actuating device of the pitch joint assembly to be the gravitational torque of the load of the distal end of the pitch joint assembly, at the same time, by adjusting the output torque of the actuating device of the yaw joint assembly to be the gravitational torque of the load of the distal end of the yaw joint assembly, an effective buffering effect for the external force can be achieved.

In some embodiments, the first joint assembly of the operating device may also not be configured by the system or the user, but determined by the processor according to the joint assembly which receives the external force. That is, the changes of a force bearing point may have an effect on the selection of the first joint assembly by the processor. The joint assembly which has a buffering effect for the external force generally includes the joint assembly where the force bearing point is located and/or the joint assembly which is in proximity to a proximal end of the joint assembly where the force bearing point is located. In this case, the joint assembly where the force bearing point is located may be referred to as the joint assembly receiving the external force.

For example, the first joint assembly may include at least one component as below: the joint assembly where the force bearing point is located, and the joint assembly which is in proximity to the proximal end of the joint assembly where the force bearing point is located. For example, all of the joint assembly where the force bearing point is located and the joint assemblies which are in proximity to the proximal end of the joint assembly where the force bearing point is located may be configured as the first joint assemblies according to the pre-configured configuration strategy. For another example, the joint assembly where the force bearing point is located may be configured as the first joint assembly according to the pre-configured configuration strategy. For another example, all of the joint assemblies which are in proximity to the proximal end of the joint assembly where the force bearing point is located may be configured as the first joint assemblies according to the pre-configured configuration strategy.

For example, the first joint assembly may include at least one component as below: the joint assembly where the force bearing point is located, and the joint assembly which is in proximity to the proximal end of the joint assembly where the force bearing point is located. At the same time, the first joint assemblies may further include the joint assemblies which are in proximity to the distal end of the joint assembly where the force bearing point is located. In this case, as the joint assembly which is in proximity to the distal end of the joint assembly where the force bearing point is located may have no effective buffering effect for the external force when receiving the external force, so that it can be determined whether to configure the joint assembly which is in proximity to the distal end of the joint assembly where the force bearing point is located to be the first joint assembly.

In this case, the joint assembly where the force bearing point is located, and the joint assembly which is in proximity to the proximal end of the joint assembly where the force bearing point is located, and the joint assembly which is in proximity to the distal end of the joint assembly where the force bearing point is located may respectively include more than one joint assemblies.

In some embodiments, whichever way the first joint assembly is determined, the joint assemblies corresponding to the DOFs of the operating device as many as possible can be configured to be the first joint assemblies. When the first joint assemblies include a plurality of joint assemblies and have no redundant DOF, the received external force can be buffered from a multiple directions. When the first joint assemblies include a plurality of joint assemblies and have at least one redundant DOF, a multi-level buffering can be achieved by using the joint assemblies corresponding to the at least one redundant DOF for the received external force from the at least one direction corresponding to the at least one redundant DOF.

In some embodiments, the output torque of the actuating device corresponding to the first joint assembly includes an output resultant torque. The output resultant torque includes a first torque and a second torque, for example, the output torque includes a sum of the first torque and the second torque. In one embodiment, the first torque may be configured for balancing the gravitational torque of the load of distal end of the first joint assembly. The second torque may be configured for restoring the joint position of the first joint assembly, or, the second torque may be configured for braking the first joint assembly.

In some embodiments, the processor may also be configured for: determining whether the operating device receives the external force.

The present disclosure may use in a variety of ways, alone or in combination, to determine whether the operating device receives the external force.

In one embodiment, the processor may be configured for: receiving current signals of an actuating device detected by a sensor, and determining whether the operating device receives the external force, and/or, determining the corresponding joint assembly where the force bearing point is located when the operating device receives the external force (that is, determining the joint assembly receiving the external force in the operating device) according to the changes of the current signals.

In the embodiment, the sensor is specifically configured for detecting the current signals at intervals of a first cycle. The processor is specifically configured for calculating a rate of change of the detected adjacent current signals, and determining whether the rate of change exceeds a first threshold, and determining that the operating device receives the external force in response to that the rate of change exceeds the first threshold, and/or, determining the joint assembly which receives the external force according to the joint assembly corresponding to the current signals of which the rate of change exceeds the first threshold. It can be understood that in response to that the rate of change does not exceed the first threshold, whether the operating device receives the external force cannot be determined temporarily, and it cannot be determined temporarily the joint assembly which receives the external force according to the joint assembly corresponding to the current signals of which the rate of change exceeds the first threshold, either.

In the embodiment, the sensor is specifically configured for detecting the current signals at intervals of a second cycle. The second cycle is larger than the first cycle. The processor is specifically configured for: calculating the rate of change of the detected adjacent current signals, determining whether the rate of change exceeds a second threshold which is smaller than the first threshold, and determining that the operating device receives the external force in response to that the rate of change exceeds the second threshold, and/or, determining the joint assembly which receives the external force according to the joint assembly corresponding to the current signals of which the rate of change exceeds the second threshold. It can be understood that in response to that the rate of change does not exceed the second threshold, whether the operating device receives the external force cannot be determined temporarily, and it cannot be determined temporarily the joint assembly which receives the external force according to the joint assembly corresponding to the current signals of which the rate of change exceeds the second threshold, either.

For example, assuming the first cycle is 1 ms, in response to that the rate of change corresponding to the first cycle exceeds the first threshold, it can be determined that the operating device receives the external force, and the joint assembly which receives the external force can also be determined.

When the rate of change of the current signals is lower, but the variation of the final current signals with the accumulation of time is higher, in fact, it can also be considered that the operating device receives the external force. Under this condition, for example, if it is otherwise directly concluded that operating device does not receive the external force based on that the rate of change corresponding to the first cycle does not exceed the threshold, then an incorrect determination result may occur, leading to a failure to buffer the external force.

And then, at the same time, the sensor may also detect the current signals at intervals of the second cycle. The second cycle may be, for example, 100 ms, in response to that the rate of change corresponding to the second cycle exceeds the second threshold which is smaller than the first threshold, then it can be determined that the operating device receives the external force, and the joint assembly which receives the external force can also be determined.

Similar to the principle, the sensor may also detect the current signals at intervals of a third cycle and even more cycle, and the thresholds of the rates of change corresponding to different cycles are different, in which, the longer the cycle, the smaller the corresponding threshold may be configured to be. In response to that the rate of change corresponding to any cycle exceeds the corresponding threshold, it can be determined that the operating device receives the external force, and the joint assembly which receives the external force can also be determined.

In the embodiment, the processor is specifically configured for calculating a current difference between the current signals and the current signals detected before the collision, and determining whether the current difference exceeds a third threshold, and determining that the operating device receives the external force in response to that the current difference exceeds the third threshold, and/or, determining the joint assembly which receives the external force according to the joint assembly corresponding to the current signals of which the current difference exceeds the third threshold. It can be understood that in response to that the current difference does not exceed the third threshold, whether the operating device receives the external force cannot be determined temporarily, and it cannot be determined temporarily joint assembly which receives the external force according to the joint assembly corresponding to the current signals of which the current difference exceeds the third threshold, either.

The way to determine whether the operating device receives the external force, and/or, determine the joint assembly which receives the external force according to whether the current difference exceeds the corresponding threshold, can be used in combination with the way to determine whether the operating device receives the external force, and/or, determine the joint assembly which receives the external force according to whether the rate of change of the detected current signals exceeds the corresponding threshold, so as to determine whether the operating device receives the external force and/or determine the joint assembly which receives the external force more accurately. In this case, in response to that any of these determination results exceeds the corresponding threshold, it can be determined that the operating device receives the external force, and the joint assembly which receives the external force can also be determined. And then, in response to that none of these determination results does not exceed the corresponding threshold, whether the operating device receives the external force cannot be determined temporarily, and the joint assembly which receives the external force cannot be determined temporarily, either.

In fact, the force bearing point of the operating device may not be limited to only one. Even if the force bearing points are more than two, the joint assemblies where the force bearing points are located can also be determined according to the above embodiments, and even if there are a plurality of the force bearing points, an effective buffering effect for the external force can also be achieved according to the above embodiments of the present disclosure.

In one embodiment, the processor is specifically configured for: receiving a force signal of the joint assembly detected by a sensor, and determining whether the operating device receives the external force, and/or, determining the joint assembly which receives the external force according to a change of the force signal.

In the embodiment, the processor is specifically configured for: determining whether the force signal is higher than a configured threshold; in response to that a detected force signal is higher than the threshold, then determining that the operating device receives the external force, otherwise, determining that the operating device does not receive the external force; and optionally determining the joint assembly which receives the external force. In this case, the joint assembly which receives the external force may be the joint assembly corresponding to the sensor whose detected force signal is higher than the threshold.

In the above embodiments, for the case that the first joint assembly is the pre-configured joint assembly, generally, it is not required to determine the joint assembly where the force bearing point is located, and it is only required to determine whether the operating device receives the external force. But for the case that the determination of the first joint assembly is associated with the location of the force bearing point, it is required to determine whether the operating device receives the external force, and it is also required to determine which joint assembly or which joint assemblies receives/receive the external force.

In the above embodiments, for the case that the first joint assembly is the pre-configured joint assembly, the processed current signals at least include the current signals of the actuating device corresponding to the first joint assembly, for example, only include the current signals of the actuating device corresponding to the first joint assembly, and/or, the processed force signal at least includes the force signal of the first joint assembly, for example, only includes the force signal of the first joint assembly.

In some embodiments, the processor may also be configured for: obtaining joint positions of at least the first joint assembly and a joint assembly which is located at the distal end of the first joint assembly of the operating device which are detected by the sensors, and determining a desired output first torque of the actuating device corresponding to the first joint assembly by a combination with the joint positions and a dynamics model associated with the first joint assembly.

The dynamics model required to be used in the present disclosure is generally constructed for the first joint assembly. For example, for the different first joint assemblies, the constructed dynamics models are different. Generally, the dynamics model corresponding to the first joint assembly is associated with the first joint assembly and the joint assembly located at the distal end of the first joint assembly of the operating device. For example, when the pitch joint assembly and the yaw joint assembly of the operating device 200' are both configured as the first joint assemblies, then the pitch joint assembly corresponds to one dynamics model, and the yaw joint assembly corresponds to another dynamics model. Assuming that the yaw joint assembly is located at the proximal end of the pitch joint assembly, then the dynamics model of the pitch joint assembly is only associated with the pitch joint assembly and the joint assembly which is located at the distal end of the pitch joint assembly, and the dynamics model of the yaw joint assembly includes the yaw joint assembly and all the joint assemblies which are associated with the dynamics model of the pitch joint assembly.

In one embodiment, the dynamics model corresponding to the first joint assembly of the operating device can be constructed as follows:

Linkage parameters of the first joint assembly and the joint assembly located at the distal end of the first joint assembly are obtained, and a coordinate system of linkage is built according to the linkage parameters. In this case, the joint assembly includes a joint and a linkage connected to the joint, the linkage parameters (that is, DH parameters) may include parameters, such as, at least one of the joint angle and the joint displacement, the linkage length, and the like.

The first dynamics model corresponding to the first joint assembly is constructed according to the coordinate system of linkage. In this case, the first dynamics model is generally in a symbolic form (that is, a formula with unknown parameters), the first dynamics model is a fuzzy (that is, the parameters of the dynamics are undetermined temporarily) dynamics model. For example, the first dynamics model may be expressed as the following formula:

$$\tau = M(\theta)\ddot{\theta} + H(\theta,\dot{\theta}) + G(\theta) \qquad \text{Formula (1),}$$

in which, $\tau$ is an actual torque of the joint assembly, $\theta$ is the joint position of the joint assembly, $\dot{\theta}$ is a speed of the joint assembly ($\dot{\theta}$ is the first derivative of $\theta$), $\ddot{\theta}$ is the speed of the joint assembly ($\ddot{\theta}$ is the second derivative of $\theta$), $M(\theta)$ is an inertia matrix, $H(\theta, \dot{\theta})$ includes a Koch force and a centrifugal force, and $G(\theta)$ is the gravitational torque of the joint assembly.

Unknown dynamics parameters of the first dynamics model are determined. In this case, the first dynamics model generally includes at least one unknown dynamics parameters, the unknown dynamics parameters associated with the formula (1) can be determined respectively, to obtain the exactly second dynamics model. In one embodiment, the contribution for the joint torque by the parts of the unknown dynamics parameters can be ignored according to the actual situation, for example, a few of the key dynamics parameters such as the mass of the joint assembly, the barycenter of the joint assembly and the friction torque of the joint assembly can be mainly focused. In some embodiments, the mass, the barycenter and the friction torque of the joint assembly may be affected by the actuating device which actuating the joint assembly and/or the transmission device which is connected between the actuating device and the joint assembly for transmitting. For example, when the structure of the operating device is relatively regular, at least parts of the dynamics parameters, such as the mass, the barycenter and the friction torque of the joint assembly can be obtained directly without identification. It can be understood that at least parts of the dynamics parameters, such as the mass, barycenter and the friction torque of the joint assembly can also be obtained by identifying. For example, the mass of the joint assembly can be obtained by weighing, the barycenter and the friction torque of the joint assembly can be obtained by identifying. For example, in some embodiments, the contribution for the joint torque by $M(\theta)$ and $H(\theta, \dot{\theta})$ can be ignored in some embodiments of the present disclosure, so that, the Formula (1) can be simplified as:

$$\tau = G(\theta) \qquad \text{Formula (2).}$$

A second dynamics model is obtained by bringing the dynamics parameters into the first dynamics model. In this case, the second dynamics model is a clear (that is, the dynamics parameters are known) dynamics model. And then, when combining the joint position and the dynamics model corresponding to the first joint assembly to determine a desired output first torque of the actuating device corresponding to the first joint assembly, the used dynamics model is the second dynamics model.

In one embodiment, considering the adverse effects of the friction torque, the friction torque can be excluded from the actual torque of the joint assembly, specifically:

The torque equilibrium model can be constructed based on a dynamic equilibrium principle. The torque equilibrium model may be expressed as the following formula:

$$\tau = k_1 * \sin\theta + k_2 * \cos\theta + f * sign(\dot{\theta}) \qquad \text{Formula (3),}$$

in which, $\tau$ is the actual torque of the joint assembly, $\theta$ is the joint position of the joint assembly, $\dot{\theta}$ is the speed of the joint assembly, $k_1$ and $k_2$ are the gravitational torque parameters, $f$ is the friction torque of the joint assembly, and $sign(\dot{\theta})$ is the direction of the speed.

The friction torque of the joint assembly can be determined by identifying. For example, the individual joint assembly can be controlled to perform a uniform motion at a low speed, and to traverse the entire range of motion, and to capture the actual torque and the corresponding joint position of the joint assembly. The individual joint assembly is the joint assembly corresponding to the first joint assembly. In this case, during the procedure of the uniform motion, the friction torque is approximately invariant and is considered to be a constant value, and then, the friction torque of the joint assembly can be identified by using the methods such as the least square method according to the captured actual torque and the corresponding joint position of the joint assembly. Understandably, the actual torque of the joint assembly is outputted by the actuating device which actuates the joint assembly.

When determining the unknown dynamics parameters (such as the gravitational torque G($\theta$) in the Formula (2)) in the first dynamics model by identifying, each of the joint assemblies can be controlled to perform a uniform motion at a low speed, and to traverse the entire range of motion, and to capture the actual torque of the first joint assembly, and to capture the corresponding joint positions of the first joint assembly and the joint assembly which is located at the distal end of the first joint assembly. The unknown dynamics parameters (such as the gravitational torque G($\theta$) in the Formula (2)) can be identified by using the methods such as the least square method by combination the actual torque of the first joint assembly, the friction torque of the first joint assembly, and the joint positions of the first joint assembly and the joint assembly which is located at the distal end of the first joint assembly. For example, in the Formula (2), the identified unknown dynamics parameters mainly include the gravitational torque parameters (including the mass, the barycenter and the like.), thus, the second dynamics model which is associated with the relationship between the joint positions and the first torque can be effectively constructed, in which, the joint positions include the joint position of the first joint assembly and joint position of the joint assembly which is located at the first joint assembly, the first torque include the first torque of the first joint assembly.

In some embodiments, the second torque of the output torque is configured for restoring the joint position of the first joint assembly of the operating device, to perform the effect like a reset spring, and to facilitate the first joint assembly to restore the joint position after the movement under the received external force, not only is the corresponding filiform structures effectively prevented from being pulled off, but also the position and/or the orientation of the operating device can be maintained in view of a final effect. For example, the operating device can be restored to the orientation before receiving the external force by outputting the second torque when the operating device receives the external force that includes a change in the orientation of the operating device, it is extremely beneficial for some stages of operation of the surgical robot. For example, assuming the condition indicating that the doctor is in the unreadiness state includes the condition that the doctor is not observing the display of the display device, such as the head of the doctor is not in proximity to the observation window.

When the doctor is in the unreadiness state, the doctor can control the surgical instrument of the slave operating device by manipulating the operating device. For intuitive control by the doctor, before the doctor controls the surgical instrument by manipulating the operating device, generally, it is required to adjust the orientation of the operating device to maintain the same with the orientation of the end effector of the surgical instrument firstly. During the procedures where the doctor normally controls the surgical instruments by manipulating the operating device, when the doctor's readiness state changes, for example, the head of the doctor changes from proximity to the observation window to be away from the observation window, at the moment of the doctor's readiness state changes, actually, the orientation of the operating device is also maintained the same with the orientation of the end effector. The orientation of the operating device can be configured as a first orientation at the moment of the doctor's readiness state changes. When the doctor is in the unreadiness state, if the operating device receives the external force that includes a change in the orientation of the operating device, the corresponding joint assembly of the operating device will be in motion to make the orientation of the operating device to be a second orientation that causes the operating device cannot maintain the same with the orientation of the end effector, and when the external force disappears, the corresponding joint assembly of the operating device will restore from the second orientation to the first orientation under the effect of the second torque to re-align with the orientation of the end effector. When the doctor's readiness state changes again, that is, when changing from the condition where the doctor is in the unreadiness state to a condition where the doctor is in readiness state, due to the orientation of the operating device is already re-align with the orientation of the end effector, it is not necessary for the processor to automatically adjust or for the doctor to manually adjust the orientation of the operating device to align with the orientation of the end effector, instead, the doctor can manipulate the operating device directly to continue to control the surgical instrument, which saves time for the operation.

In some embodiments, the processor is configured for:

obtaining an initial joint position and a current joint position of the first joint assembly detected by a sensor; calculating a joint position variable of the first joint assembly based on the initial joint position and the current joint position; and determining the second torque outputted by the actuating device corresponding to the first joint assembly based on the joint position variable. In this case, the initial joint position includes a joint position before receiving the external force.

Preferably, the second torque increases as the joint position variable increases, that is, the larger the joint position variable, the larger the restoring force; and the smaller the joint position variable, the smaller the restoring force. More preferably, the second torque increases linearly as the joint position variable increases.

For example, the relationship between the second torque and the joint position variable can be expressed as below:

$$\tau = k * \Delta x \qquad \text{Formula (4),}$$

in which, $\tau$ is the second torque, $k$ is a stiffness coefficient, and $\Delta x$ is the joint position variable.

In some embodiments, the second torque may also be configured for braking the motion of the first joint assembly of the operating device under the effect of the external force, and therefore may function like a brake, to facilitate braking of the first joint assembly under the collisions. The processor may be configured for:

obtaining the current joint position of the first joint assembly detected by the sensor; determining whether the current joint position reaches a first limiting joint position of the first joint assembly, in which, the first limiting joint position is within a range of a physical limiting joint position of the first joint assembly; in response to that the current joint position reaches the first limiting joint position, calculating a joint position variable between the current joint position and the first limiting joint position; and determining the second torque outputted by the actuating device corresponding to the first joint assembly based on the joint position variable.

Preferably, the second torque increases as the joint position variable increases. More preferably, the second torque increases linearly as the joint position variable increases. For example, the Formula (4) can also be used for realizing braking, which is not repeated herein. In this case, the first joint assembly can move freely under the effect of the external force in response to that the first joint assembly does not reach the first joint position, and the restoring force for resisting the first joint assembly to move to the physical limiting position to realize braking can be generated from the first joint position in response to that the first joint assembly reaches the first joint position. During the non-braking stage, the first joint assembly can be avoided quickly under the external force, and during the braking stage, the first joint assembly can be braked progressively by using the spring principle to avoid the unexpected problems caused by the rapid braking.

In one embodiment, when the second torque is the braking torque, the processor may also be configured for:

obtaining an initial joint position and a current joint position of the first joint assembly detected by a sensor; calculating a joint speed (that is, joint velocity) of the first joint assembly based on the initial joint position, the current joint position, and a time during which the initial joint position changes to the current joint position; and determining the second torque outputted by the actuating device corresponding to the first joint assembly based on the joint speed.

Preferably, the second torque increases as the joint speed increases, that is, the faster the joint speed, the larger the braking force; and the slower the joint speed, the smaller the braking force. For example, the relationship between the second torque and the joint speed can be expressed as below:

$$\tau = k * v^n \qquad \text{Formula (5),}$$

in which, $\tau$ is the second torque, $k$ is a damping coefficient, $v$ is the joint speed, n is an integer greater than or equal to one, for example, n = 1, or n = 2, or n = 3. In this case, when n = 1, the braking effect is more linear, and when n = 2, or n = 3, compared to the case where n = 1, the braking is quicker and more agile.

In the above embodiments, the processor can adjust the output torque of the first joint assembly according to the output resultant torque which is the sum of the determined first torque and the determined second torque, and a good effect associated with the way the second torque acquired can be achieved.

In one embodiment, for example, in the case that the second torque uses a principle of a spring model, that is, using the Formula (4) to realize restoring the joint position of the first joint assembly or braking the motion of the first joint assembly, the processor may also be configured for: recording a holding time of the first joint assembly at the current joint position during a duration of the operating device receiving the external force, and updating the current joint position to be the initial joint position in response to that the holding time reaches a configured time threshold. In the embodiment, it can be recorded that the holding time of the first joint assembly at the current joint position begins at the time when it is determined that the operating device receives the external force. And/or, the processor may also be configured for: obtaining the current joint position of the first joint assembly during the duration of the operating device receiving the external force, recording a first holding time in which a variable of the current joint position is lower than a position variation threshold in response to that the variable of the current joint position is lower than the position variation threshold, and updating the current joint position to be the initial joint position in response to that the first holding time reaches a first configured time threshold. For the rotary joint assembly of the first joint assembly, the range of the position variation threshold may include 0 - 0.1°, for example, the position variation threshold may be configured to be 0.01°. For the displacement joint assembly of the first joint assembly, the range of the position variation threshold may include 0 - 0.1 mm, for example, the position variation threshold may be configured to be 0.01 mm. And/or, the processor may also be configured for: obtaining a joint speed of the first joint assembly during a duration of the operating device receiving the external force, recording a second holding time in which the joint speed is lower than a speed threshold in response to that the joint speed is lower than the speed threshold, and updating the current joint position to be the initial joint position in response to that the second holding time reaches a second configured time threshold. For the rotary joint assembly of the first joint assembly, the range of the speed threshold may include 0 - 0.1°/s, for example, the speed threshold may be configured to be 0.0001°/s. For the displacement joint assembly of the first joint assembly, the range of the speed threshold may include 0-0.01 mm/s, for example, the speed threshold may be configured to be 0.0001 mm/s. The configured time threshold, the first configured time threshold, and the second configured time threshold can be exactly the same, partially the same or completely different, for example, all can be configured as 4-8 seconds, for example, all can be configured as 6 seconds. For example, the first joint assemblies may include the whole or the partial of the joint assemblies which are configured to be the first joint assemblies, for example, when all the joint assemblies of the first joint assemblies satisfy any of the above conditions, the current joint position is updated to be the initial joint position. In the embodiment, the initial joint positions are updated and no longer the joint positions before the operating device receives the external force. In such condition, exiting the impedance mode can be realized, and the impedance mode can be re-entered only when it is determined that the operating device receives an external force again. The impedance mode includes: when it is determined that the operating device receives the external force, in response to the external force received by the operating device, adjusting the output torque of the actuating device corresponding to the first joint assembly of the joint assemblies to balance the gravitational torque of the load of the distal end of the first joint assembly, and to restore the joint position of the first joint assembly or brake the first joint assembly. In addition, the duration in which the operating device receives the external force includes the entire period from the determination of the operating device receiving the external force to the exiting of the impedance mode. During the duration in which the operating device receives the external force, the output torque of the actuating device corresponding to the first joint assembly is required to balance the gravitational torque of the load of the distal end of the corresponding first joint assembly, and to restore the joint position of the first joint assembly or to brake the first joint assembly.

In the embodiment, the joint position variable of the first joint assembly may be re-calculated based on the updated initial joint position and the current position, so that the first torque can change periodically, and the operating device can behave as a state of a staged motion.

The present disclosure may also provide a control method for the surgical robot, the control method may include any one of steps executed and implemented by the processor as described in the above, which will not be repeated herein.

Continue to FIG. 2, the arm of the slave operating device 100 can be divided into three parts, for example, each of the parts can be defined to a kind of sub-arms, correspondingly. The three kinds of sub-arms include a main arm 110, an adjusting arm 120, and a manipulator 130 which are sequentially connected. As illustrated in FIG. 1, the number of the main arm 110 may be one, the number of the adjusting arm 120 may be more than two, for example, and the number of the adjusting arm 120 may be four as illustrated in FIG. 1. The number of the manipulators 130 may be the same with the number of the adjusting arms 120. In this case, a proximal end of the main arm 110 may be arranged on the movable base 300, a distal end of the main arm 110 may be provided with an orienting platform, a proximal end of each of the adjusting arms 120 may be rotatably connected to the orienting platform, and a distal end of each of the adjusting arms 120 may be rotatably connected to one of the manipulators 130. The main arm 110 may be mainly used for a primary location planning for the whole of the manipulators 130, and has the characteristics of holistic, relatively large in scope and relatively low in precision. The adjusting arm 120 may be used for a secondary location planning for the manipulator 130 connected thereto, and has the characteristics of independent, relatively small in scope and relatively high in precision. The manipulator 130 is detachably connected to a device configured for operation, which is the surgical instrument 150, the surgical instruments 150 may include an imaging instrument and an operating instrument. As the surgical instrument 150 may also include a joint assembly that may affect the position and/or the orientation of the end effector which is located at the distal end of the joint assembly, the surgical instrument 150 can be considered to be one kind of sub-arms. Referring to FIG. 1, in the slave operating device 100, along the direction of the base 300 towards the surgical instrument 150, the "distal end" indicates the surgical instrument 150 side, the "proximal end" indicates the base 300 side, the surgical instrument 150 is relatively in proximity to the patient, and the base 300 is relatively away from the patient.

Continue to FIG. 1, the distal end of the manipulator 130 may include an instrument arm 135, the instrument arm 135 may be configured for detachably mounting with the surgical instrument 150, and the instrument arm 135 restricts a remote center of motion (that is, the remote center, RC) of the manipulator 130. The manipulator 130 uses the principle of the parallelogram in such a way that the manipulator 130 can rotate around the remote center of motion. The surgical instrument 150 can follow the manipulator 130 to rotate around the remote center of motion, and the surgical instrument 150 can move in depth (that is, feed) relative to the instrument arm 135. Referring to FIG. 1, the manipulator 130 may also include the pitch joint assembly 131 and the yaw joint assembly 132, when actuating the actuating device corresponding to the pitch joint assembly 131 to work, the manipulator 130 can rotate around the remote center of motion in a pitching motion, and when actuating the actuating device corresponding to the yaw joint assembly 132 to work, the manipulator 130 can rotate around the remote center of motion in a yawing motion.

Each sub-arm may also include actuating devices which are coupled to the corresponding joint assemblies of each sub-arm and configured for actuating the corresponding joint assemblies, respectively, generally, each joint assembly actuated by a corresponding actuating device. In these sub-arms, each actuating device can be configured to be arranged adjacent to the joint assembly which is actuated by the actuating device. And/or, the actuating device can be configured to be arranged away from the joint assembly which is actuated by the actuating device. For example, in the main arm 110, the adjusting arm 120 and the manipulator 130, as they have sufficient space, generally, the actuating device and the joint assembly can be arranged adjacent to each other. But in the surgical instrument 150, with the need for the compact structure, generally, the actuating device and the joint assembly can be arranged away from each other and driven by means of the driving wires. For example, the actuating devices which actuate the joint assemblies of the surgical instrument 150 are arranged at the distal end of the manipulator 130, for example, arranged at the instrument arm 135. For realizing the couple connection between the remotely arranged actuating devices and the joint assemblies of the surgical instrument 150, the surgical instrument 150 may include an actuating box coupled to the joint assemblies, through an adapter which may be detachably connected to the remotely arranged actuating devices and the actuating box, the surgical instrument 150 can be attached to the instrument arm 135 and the remotely arranged actuating devices and the joint assemblies of the surgical instrument 150 can be connected.

In the surgical robot, specifically, in the slave operating device 100, multiple kinds of sensors may also be included, and these sensors are respectively coupled to the processor. In these sensors are included sensors for detecting (that is, sensing) the joint position of the joint assemblies of the arms of the slave operating device 100, the sensor for such use may be, for example, a position sensor for detecting the joint position of the joint assemblies of the arms of the slave operating device 100, and may also be at least one of the encoder and the position sensor for detecting the position of the actuating devices which actuate the joint assemblies of the arms of the slave operating device 100. And/or, in these sensors, are included sensors for detecting the current signals of the actuating devices of the arms of the slave operating device 100 for actuating the joint assemblies to move, the sensor for the use may be such as at least one of the current sensor and the voltage sensor. And/or, in these sensors, are included sensors for detecting the force signal of the joint assemblies of the arms of the slave operating device 100, the sensor for the use may be such as at least one of the three-dimensional force sensor, the six-dimensional force sensor, and a combination of multiple of the one-dimensional force sensors.

In the above embodiments, for example, the actuating devices of the operating device 210' and the slave operating device 100 may include motors, generally, the corresponding joint assemblies can be actuated by controlling the motors to work.

In one aspect, a first arm described in the present disclosure can consist of at least part of the joint assemblies of one or more sub-arms. The first arm not only applies to the slave operating device which includes the joint assemblies and the actuating devices, but also applies to the operating device which includes the joint assemblies and the actuating devices.

In some embodiments, as an example, the operating device 200' may be illustrated as follows:

For example, any of the sub-arms can be configured as the first arm, the first arm may include all the joint assemblies of the sub-arm. For example, the master arm 230' can be configured as the first arm, and the master wrist 240' can also be configured as the first arm.

For example, the combination of at least two of the sub-arms can also be configured as the first arm, the at least two of the sub-arms constituting the first arm can be the sub-arms adjacent to each other, and the first arm may include all the joint assemblies of the sub-arms. For example, the master arm 230' and the master wrist 240' can be configured as the first arm.

For example, in these sub-arms, at least part of the joint assemblies of any one of the sub-arms and at least part of the joint assemblies of any other of the sub-arms can also be configured as the first arm. For example, all or part of the joint assemblies of one sub-arm and part or all of the joint assemblies of another sub-arm can be configured as the first arm. For example, all of the joint assemblies of the master arm 230' and part of the joint assemblies of the master wrist 240' can be configured as the first arm.

For example, part of the joint assemblies of any of the sub-arms can also be configured as the first sub-arm. For example, in the operating device as illustrated in FIG. 3, part of the joint assemblies of the master wrist 240' can be configured as the first arm. In this case, the master wrist 240' may include at least one joint assembly of the yaw DOF, the pitch DOF and the roll DOF that are used for manipulating the end effector of the surgical instrument 150 of the slave operating device.

In some embodiments, the specific constitution of the first arm can be configured by the system, and can also be configured by the user flexibly.

In some embodiments, as an example, the slave operating device 100 may be illustrated as follows:

For example, any of the sub-arms can be configured as the first arm, the first arm may include all the joint assemblies of the sub-arm. For example, in the surgical robot as illustrated in FIG. 1, the main arm 110 can be configured as the first arm, or the adjusting arm 120 can be configured as the first arm, or the manipulator 130 can be configured as the first arm, or the surgical instrument 150 can also be configured as the first arm.

For example, the combination of at least two of the sub-arms can also be configured as the first arm, the at least two of the sub-arms constituting the first arm can be the sub-arms adjacent to each other, or can also be the sub-arms spacedly arranged. The first arm may include all the joint assemblies of the sub-arms. For example, in the surgical robot as illustrated in FIG. 1, the main arm 110 and any of the adjusting arms 120 can be configured as the first arm, or any of the adjusting arm 120 and the manipulator 130 connected thereto can be configured as the first arm, or the adjusting arm 120, the manipulator 130 connected to the adjusting arm 120 and the surgical instrument 150 connected to the manipulator 130 can be configured as the first arm, or the adjusting arm 120 and the corresponding surgical instrument 150 can also be configured as the first arm, which will not be specifically listed herein.

For example, in these sub-arms, at least part of the joint assemblies of any of the sub-arms and at least part of the joint assemblies of any other of the sub-arms can also be configured as the first arm. For example, all or part of the joint assemblies of one sub-arm and part or all the joint assemblies of another sub-arm can be configured as the first arm. For example, the joint assembly 121 such as the tornado rotational joint assembly located at the distal end of the adjusting arm 120 and all the joint assemblies of the manipulator 130 can be configured as the first arm, in which, the rotation axis of the tornado rotational joint assembly 121 and the remote center of motion are intersected, so that the manipulator 130 can still rotate around the remote center of motion while the tornado rotational joint assembly 121 rotates.

For example, part of the joint assemblies of any of the sub-arms can also be configured as the first sub-arm. For example, the pitch joint assembly 131 and the yaw joint assembly 132 of the manipulator 130 can be configured as the first arm.

In some embodiments, the specific constitution of the first arm can be configured by the system, or can also be configured by the user flexibly. For example, the surgical robot includes a display device and an input device that are respectively electrically connected to the processor. The first arm can be configured as follows: a user configuration interface is generated by the processor and displayed on the display device, and in turn selection may be made by means of the input device. The display device and the input device can be a touch screen, and can also be two separate components. In the user configuration interface, the options associated with each sub-arm can be generated. In the user configuration interface, the options associated with the joint assemblies of each sub-arm can be generated. In this case, the options associated with the sub-arms and/or the joint assemblies of each sub-arm may be the graphical options, or may also be the characterized (such as textualisation, or digitalization) options. It may configure the compositions of the first arm by selecting these options.

In another aspect, the first arm may include at least one arm not being operated during the collision. For example, at least during the collision, some arm and/or a device attached to the distal end of the arm is not in case of a following state, such arm may be the arm not being operated, and may be used as the first arm. For example, at least during the collision, some arm and/or a device attached to the distal end of the arm is in a clutch state, that is, the motion input device 210 is in case of a disconnect communication, such arm may be the arm not being operated, and may be used as the first arm. In this case, when the arm is some sub-arm, the device attached to the distal end of the sub-arm may include the other sub-arm and/or the surgical instrument 150.

In some embodiments, the processor may be configured for: determining whether the first arm and/or the device attached to the distal end of the first arm collides, when it is determined that the first arm and/or the device attached to the distal end of the first arm collides, in response to the collision of the first arm and/or the device attached to the distal end of the first arm, adjusting an output torque of an actuating device corresponding to a first joint assembly of the joint assemblies of the first arm for balancing a gravitational torque generated by a load of a distal end of the first joint assembly, and to restore a joint position of the first joint assembly or brake the first joint assembly.

The "balance" described in the present disclosure generally means a relatively balanced relationship, which allows the presence of the torque deviation. For a same first joint assembly, the balance may be considered to be realized when the following formula is satisfied:

$$x = \left| F_1 - F_2 \right| \le \Delta x ,$$

x is the torque deviation, F1 is the output torque of the actuating device, F2 is the gravitational torque of the load of the distal end of the corresponding first joint assembly,
Δx is the torque deviation threshold. For example, the range of values of Δx may be [0, 10] N/m.

In this case, as long as $x \le \Delta x$, the output torque of the actuating device can be considered to balance the gravitational torque of the load of the distal end of the corresponding joint assembly.

And then, when the first arm is collided, the adjusted joint assembly of the first arm, which is the first joint assembly, can be deflected and/or moved easily under the effect of the external force, to effectively buffer the external force by the changes in position and/or in orientation.

In some embodiments, the first joint assembly of the first arm may be formed by a joint assembly which is configured by the system or the user. In the embodiments, which joint assembly of the first arm is exactly included by the first joint assembly is pre-determined (that is, pre-configured) before the collision. For example, the first joint assembly of the first arm can be obtained from the system files, and the first joint assembly of the first arm may also be configured by the user configuration interface. In this case, the first joint assembly generally means a type of joint assembly, and the number of the first joint assembly includes at least one. For example, when the first joint assemblies include at least two joint assemblies, the processor can be configured to adjust the output torque of the actuating device corresponding to each first joint assembly to balance the gravitational torque of the load of the distal end of the corresponding first joint assembly respectively.

For example, in the slave operating device as illustrated in FIG. 1, the manipulator 130 can be configured as the first arm. Optionally, for example, both of the pitch joint assembly 131 and the yaw joint assembly 132 of the manipulator 130 can be configured as the first joint assemblies of the first arm. In this example, if the force caused by the collision has component forces in the pitch DOF corresponding to the pitch joint assembly 131 and/or in the yaw DOF corresponding to the yaw joint assembly 132, by adjusting the output torque of the actuating device of the pitch joint assembly 131 to be the gravitational torque of the load of the distal end of the pitch joint assembly 131, at the same time, by adjusting the output torque of the actuating device of the yaw joint assembly 132 to be the gravitational torque of the load of the distal end of the yaw joint assembly 132, an effective buffering effect for the collision can be achieved.

In some embodiments, the first joint assembly of the first arm may also not be completely configured by the system or the user, but determined based on the position of the crash point located at the first arm and/or the device attached to the distal end of the first arm, which is relatively more flexible, that is, in some extent, the changes in position of the crash point may affect the determination of the first joint assembly. In this case, the joint assembly having a buffering effect for the collision is generally a joint assembly where the crash point is located and/or a joint assembly which is in proximity to the proximal end of the j oint assembly where the crash point is located.

For example, the first joint assembly may include at least one component as below: the joint assembly where the crash point is located, and the joint assembly which is in proximity to the proximal end of the joint assembly where the crash point is located. For example, all of the joint assembly where the crash point is located and the joint assemblies which are in proximity to the proximal end of the joint assembly where the crash point is located may be configured as the first joint assemblies according to the pre-configured configuration strategy. For another example, the joint assembly where the crash point is located may be configured as the first joint assembly according to the pre-configured configuration strategy. For another example, all of the joint assemblies which are in proximity to the proximal end of the joint assembly where the crash point is located may be configured as the first joint assemblies according to the pre-configured configuration strategy.

For example, the first joint assembly may include at least one component as below: the joint assembly where the crash point is located, and the joint assembly which is in proximity to the proximal end of the j oint assembly where the crash point is located. At the same time, the first joint assemblies may further include the joint assemblies which are in proximity to the distal end of the joint assembly where the crash point is located. In this case, as the joint assembly which is in proximity to the distal end of the joint assembly where the crash point is located may have no effective buffering effect for the collision when collision occurs, so that it can be determined whether to configure the joint assembly which is in proximity to the distal end of the joint assembly where the crash point is located to be the first joint assembly.

In this case, the joint assembly where the crash point is located, and the joint assembly which is in proximity to the proximal end of the joint assembly where the crash point is located, and the joint assembly which is in proximity to the distal end of the joint assembly where the crash point is located may respectively include more than one joint assemblies.

The joint assembly where the crash point is located may include two cases. The first case is the crash point is actually located at a certain joint assembly of the first arm. The second case is the crash point is actually not located at any joint assembly of the first arm, but is located at the device attached to a distal end of the first arm, in such case, a joint assembly of the first arm which is in proximity to the device can be configured as the joint assembly where the crash point is located.

In some embodiments, whichever way the first joint assembly is determined, the joint assemblies corresponding to the DOFs of the operating device as many as possible can be configured to be the first joint assemblies. When the first joint assemblies include a plurality of joint assemblies and have no redundant DOF, the collision can be buffered from a multiple directions. When the first joint assemblies include a plurality of joint assemblies and have at least one redundant DOF, a multi-level buffering can be achieved by using the joint assemblies corresponding to the at least one redundant DOF for the collision from the at least one direction corresponding to the at least one redundant DOF.

In some embodiments, the first arm at least includes an active joint assembly, the active joint assembly refers to the joint assembly which is actuated by the actuating device. The first joint assembly generally refers to the active joint assembly.

In some embodiments, the output torque of the actuating device corresponding to the first joint assembly includes an output resultant torque. The output resultant torque includes a first torque and a second torque, for example, the output torque includes a sum of the first torque and the second torque. In one embodiment, the first torque may be configured for balancing the gravitational torque of the load of distal end of the first joint assembly. The second torque may be configured for restoring the joint position of the first joint assembly, or, the second torque may be configured for braking the first joint assembly.

In some embodiments, the "collision" described in the present disclosure may include object-to-object collision and person-to-object collision. Broadly speaking, the "collision" may also include the object receiving the external force. In this case, the "object" mainly refers to the arm in the present disclosure, such as the first arm of the arms.

The present disclosure may be used in a variety of ways, alone or in combination, to determine whether the first arm has a collision.

In one embodiment, the processor may be configured for: receiving current signals of an actuating device detected by a sensor, and determining whether the first arm and/or the device attached to the distal end of the first arm has a collision, and/or, determining the joint assembly where the crash point is located while the collision happening according to the changes of the current signals.

In the embodiment, the sensor is specifically configured for detecting the current signals at intervals of a first cycle. The processor is specifically configured for: calculating a rate of change of the detected adjacent current signals, and determining whether the rate of change exceeds a first threshold, and determining that the first arm and/or the device attached to the distal end of the first arm has the collision in response to that the rate of change exceeds the first threshold, and/or, determining the joint assembly where the crash point is located while the collision happening according to the joint assembly corresponding to the current signals of which the rate of change exceeds the first threshold. It can be understood that in response to that the rate of change does not exceed the first threshold, whether the first arm and/or the device attached to the distal end of the first arm has the collision cannot be determined temporarily, and it cannot be determined temporarily the joint assembly where the crash point is located while the collision happening according to the joint assembly corresponding to the current signals of which the rate of change exceeds the first threshold, either.

In the embodiment, the sensor is specifically configured for detecting the current signals at intervals of a second cycle, and the second cycle is larger than the first cycle. The processor is specifically configured for: calculating a rate of change of the detected adjacent current signals, and determining whether the rate of change exceeds a second threshold which is smaller than the first threshold, and determining that the first arm and/or the device attached to the distal end of the first arm has the collision in response to that the rate of change exceeds the second threshold, and/or, determining the joint assembly where the crash point is located while the collision happening according to the joint assembly corresponding to the current signals of which the rate of change exceeds the second threshold. It can be understood that in response to that the rate of change does not exceed the second threshold, whether the first arm and/or the device attached to the distal end of the first arm has the collision cannot be determined temporarily, and it cannot be determined temporarily the joint assembly where the crash point is located while the collision happening according to the joint assembly corresponding to the current signals of which the rate of change exceeds the second threshold, either.

For example, assuming the first cycle is 1 ms, in response to that the rate of change corresponding to the first cycle exceeds the first threshold, it can be determined that the first arm and/or the device attached to the distal end of the first arm has the collision, and the joint assembly where the crash point is located while the collision happening can also be determined.

When the rate of change of the current signals is lower, but the variation of the final current signals with the accumulation of time is higher, in fact, it can also be considered that the collision happens. Under this condition, for example, if it is otherwise directly concluded that the first arm and/or the device attached to the distal end of the first arm does not have the collision based on that the rate of change corresponding to the first cycle does not exceed the threshold, then an incorrect determination result may occur, leading to a failure to buffer the collision.

And then, at the same time, the sensor may also detect the current signals at intervals of the second cycle. The second cycle such as 100 ms, in response to that the rate of change corresponding to the second cycle exceeds the second threshold which is smaller than the first threshold, then it can be determined that the first arm and/or the device attached to the distal end of the first arm has the collision, and the joint assembly where the crash point is located while the collision happening can also be determined.

Similar to the principle, the sensor may also detect the current signals at intervals of a third cycle and even more cycle, and the thresholds of the rates of change corresponding to the different cycle are different, in which, the longer the cycle, the smaller the corresponding threshold may be configured to be. In response to that the rate of change corresponding to any cycle exceeds the corresponding threshold, then it can be determined that the first arm and/or the device attached to the distal end of the first arm has the collision, and the joint assembly where the crash point is located while the collision happening can also be determined.

In the embodiment, the processor is specifically configured for: calculating a current difference between the current signals and the current signals detected before the collision, and determining whether the current difference exceeds a third threshold, and determining that the first arm and/or the device attached to the distal end of the first arm has the collision in response to that the current difference exceeds the third threshold, and/or, determining the joint assembly where the crash point is located while the collision happening according to the joint assembly corresponding to the current signals of which the current difference exceeds the third threshold. It can be understood that in response to that the current difference does not exceed the third threshold, whether the first arm and/or the device attached to the distal end of the first arm has the collision cannot be determined temporarily, and it cannot be determined temporarily the joint assembly where the crash point is located while the collision happening according to the joint assembly corresponding to the current signals of which the current difference exceeds the third threshold, either.

The way to determine whether the first arm and/or the device attached to the distal end of the first arm has the collision, and/or, determine the joint assembly where the crash point is located while the collision happening according to whether the current difference exceeds the corresponding threshold, can be used in combination with the way to determine whether the first arm and/or the device attached to the distal end of the first arm has the collision, and/or, determine the joint assembly where the crash point is located while the collision happening according to whether the rate of change of the detected current signals exceeds the corresponding threshold, to determine whether the first arm and/or the device attached to the distal end of the first arm has the collision and/or determine the joint assembly where the crash point is located while the collision happening more accurately. In this case, in response to that any of these determination results exceeds the corresponding threshold, it can be determined that the first arm and/or the device attached to the distal end of the first arm has the collision, and the joint assembly where the crash point is located while the collision happening can also be determined. And then, in response to that none of these determination results does not exceed the corresponding threshold, whether the first arm and/or the device attached to the distal end of the first arm has the collision cannot be determined temporarily, and it cannot be determined temporarily the joint assembly where the crash point is located while the collision happening, either.

In fact, the crash point of the first arm may not be limited to only one. Even if the crash points are more than two, the joint assemblies where the crash points located can also be determined according to the above embodiments, and even if there are a plurality of the crash points, an effective buffering effect for the collision can also be achieved according to the above embodiments of the present disclosure.

In one embodiment, the processor is specifically configured for: receiving a force signal of the joint assembly detected by a sensor, and determining whether the first arm and/or the device attached to the distal end of the first arm has the collision, and/or, determining the joint assembly where the crash point is located while the collision happening according to a change of the force signal.

In the embodiment, the processor is specifically configured for: determining whether the force signal is higher than a configured threshold; in response to that a detected force signal is higher than the threshold, then determining that the first arm and/or the device attached to the distal end of the first arm has the collision, otherwise, determining the first arm and/or the device attached to the distal end of the first arm does not have the collision, and optionally, determining the joint assembly where the crash point is located while the collision happening. In this case, the joint assembly where the crash point is located while the collision happening may be the joint assembly corresponding to the sensor that the force signals is higher than the threshold.

In the above embodiments, for the case that the first joint assembly is the pre-configured joint assembly, generally, it is not required to determine the joint assembly where the crash point is located while the collision happening, and it is only required to determine whether the first arm and/or the device attached to the distal end of the first arm has the collision. But for the case that the determination of the first joint assembly is associated with the locations of the crash points, it is required to determine whether the first arm and/or the device attached to the distal end of the first arm has the collision, and it is also required to determine the joint assembly where the crash point is located while the collision happening.

In the above embodiments, for the case that the first joint assembly is the pre-configured joint assembly, the processed current signals at least include the current signals of the actuating device corresponding to the first joint assembly, for example, only include the current signals of the actuating device corresponding to the first joint assembly, and/or, the processed force signals at least include the force signals of the first joint assembly, for example, only include the force signals of the first joint assembly.

In another embodiment, generally, the surgical robot may also include a second arm, the second arm and the first arm generally are the different arms, and each of them is at least one part of the arms connected in parallel. The processor may be configured for: determining whether the position parameters of the kinematic model of the first arm and/or the device attached to the distal end of the first arm are the same with the position parameters of the kinematic model of the second arm and/or the device attached to the second arm, and determining whether the first arm and/or the device attached to the distal end of the first arm has a collision and/or determining the joint assembly where the crash point is located while the collision happening according to the condition whether the position parameters are the same. In this case, the "same" here may include the same and the approximately the same.

Specifically, in response to that the position parameters of the kinematic model of the first arm and/or the device attached to the distal end of the first arm are the same with (that is, coincide with) the position parameters of the kinematic model of the second arm and/or the device attached to the second arm, determining that the first arm and/or the device attached to the distal end of the first arm has the collision, and/or, determining the joint assembly where the crash point is located while the collision happening according to the joint assemblies where the same position parameters located. In response to that the position parameters of the kinematic model of the first arm and/or the device attached to the distal end of the first arm are not the same with the position parameters of the kinematic model of the second arm and/or the device attached to the second arm, determining that the first arm and/or the device attached to the distal end of the first arm does not have the collision temporarily.

In one embodiment, the processor may also be configured for: obtaining joint positions of at least the first joint assembly and a joint assembly which is located at the distal end of the first joint assembly which are detected by the sensors, and determining a desired output first torque of the actuating device corresponding to the first joint assembly by combination with the joint positions and a dynamics model associated with the first joint assembly.

The dynamics model required to be used in the present disclosure is generally constructed for the first joint assembly. For example, for the different first joint assemblies, the constructed dynamics models are different. Generally, the dynamics model corresponding to the first joint assembly is associated with the first joint assembly and the joint assembly located at the distal end of the first joint assembly of the first arm. For example, for the first arm is the manipulator 130, when the pitch joint assembly 131 and the yaw joint assembly 132 of the manipulator 130 are configured as the first joint assemblies, then the pitch joint assembly 131 is corresponding to one dynamics model, and the yaw joint assembly 132 is corresponding to another dynamics model. Assuming the yaw joint assembly 132 is located at the proximal end of the pitch joint assembly 131, then the dynamics model of the pitch joint assembly 131 is only associated with the pitch joint assembly 131 and the joint assembly which is located at the distal end of the pitch joint assembly 131, and the dynamics model of the yaw joint assembly 132 includes the yaw joint assembly 132 and all the joint assemblies which are associated with the dynamics model of the pitch joint assembly 131. Understandable, for the case that the first arm is consisted by more than two sub-arms, the corresponding dynamics model is also constructed based on the first joint assembly, which would not be repeated herein.

In one embodiment, the dynamics model corresponding to the first joint assembly can be constructed as follows:

Linkage parameters of the first joint assembly and the joint assembly located at the distal end of the first joint assembly are obtained, and a coordinate system of linkage is built according to the linkage parameters. In this case, the joint assembly includes a joint and a linkage connected to the joint, the linkage parameters (that is, DH parameters) may include parameters, such as, at least one of the joint angle and the joint displacement, the linkage length, and the like.

The first dynamics model corresponding to the first joint assembly is constructed according to the coordinate system of linkage. In this case, the first dynamics model is generally in a symbolic form (that is, a formula with unknown parameters), the first dynamics model is a fuzzy (that is, the parameters of the dynamics are undetermined temporarily) dynamics model. For example, the first dynamics model may be expressed as the following formula:

$$\tau = M(\theta)\ddot{\theta} + H(\theta, \dot{\theta}) + G(\theta) \qquad \text{Formula (1),}$$

in which, $\tau$ is an actual torque of the joint assembly, $\theta$ is the joint position of the joint assembly, $\dot{\theta}$ is a speed of the joint assembly ($\dot{\theta}$ is the first derivative of $\theta$), $\ddot{\theta}$ is the speed of the joint assembly ($\ddot{\theta}$ is the second derivative of $\theta$), $M(\theta)$ is an inertia matrix, $H(\theta, \dot{\theta})$ includes a Koch force and a centrifugal force, and $G(\theta)$ is the gravitational torque of the joint assembly.

Unknown dynamics parameters of the first dynamics model are determined. In this case, the first dynamics model generally includes at least one unknown dynamics parameters, the unknown dynamics parameters associated with the formula (1) can be determined respectively, to obtain the exactly second dynamics model. In one embodiment, the contribution for the joint torque by the parts of the unknown dynamics parameters can be ignored according to the actual situation, for example, a few of the key dynamics parameters such as the mass of the joint assembly, the barycenter of the joint assembly and the friction torque of the joint assembly can be mainly focused. In some embodiments, the mass, the barycenter and the friction torque of the joint assembly may be affected by the actuating device which actuating the joint assembly and/or the transmission device which is connected between the actuating device and the joint assembly for transmitting. For example, when the structure of the operating device is relatively regular, at least parts of the dynamics parameters, such as the mass, the barycenter and the friction torque of the joint assembly can be obtained directly without identification. It can be understood that at least parts of the dynamics parameters, such as the mass, barycenter and the friction torque of the joint assembly can also be obtained by identifying. For example, the mass of the joint assembly can be obtained by weighing, the barycenter and the friction torque of the joint assembly can be obtained by identifying. For example, in some embodiments, the contribution for the joint torque by $M(\theta)$ and $H(\theta,\dot{\theta})$ can be ignored in some embodiments of the present disclosure, so that, the Formula (1) can be simplified as:

$$\tau = G(\theta) \qquad \text{Formula (2)}.$$

A second dynamics model is obtained by bringing the dynamics parameters into the first dynamics model. In this case, the second dynamics model is a clear (that is, the dynamics parameters are known) dynamics model. And then, when combining the joint position and the dynamics model corresponding to the first joint assembly to determine a desired output first torque of the actuating device corresponding to the first joint assembly, the used dynamics model is the second dynamics model.

In one embodiment, considering the adverse effects of the friction torque, the friction torque can be excluded from the actual torque of the joint assembly, specifically:

The torque equilibrium model can be constructed based on a dynamic equilibrium principle. The torque equilibrium model may be expressed as the following formula:

$$\tau = k_1 * \sin\theta + k_2 * \cos\theta + f * sign(\dot{\theta}) \qquad \text{Formula (3)},$$

in which, $\tau$ is the actual torque of the joint assembly, $\theta$ is the joint position of the joint assembly, $\dot{\theta}$ is the speed of the joint assembly, $k_1$ and $k_2$ are the gravitational torque parameters, $f$ is the friction torque of the joint assembly, and $sign(\dot{\theta})$ is the direction of the speed.

The friction torque of the joint assembly can be determined by identifying. For example, the individual joint assembly can be controlled to perform a uniform motion at a low speed, and to traverse the entire range of motion, and to capture the actual torque and the corresponding joint position of the joint assembly. The individual joint assembly is the joint assembly corresponding to the first joint assembly. In this case, during the procedure of the uniform motion, the friction torque is approximately invariant and is considered to be a constant value, and then, the friction torque of the joint assembly can be identified by using the methods such as the least square method according to the captured actual torque and the corresponding joint position of the joint assembly. Understandably, the actual torque of the joint assembly is outputted by the actuating device which actuates the joint assembly.

When determining the unknown dynamics parameters (such as the gravitational torque $G(\theta)$ in the Formula (2)) in the first dynamics model by identifying, each of the joint assemblies can be controlled to perform a uniform motion at a low speed, and to traverse the entire range of motion, and to capture the actual torque of the first joint assembly, and to capture the corresponding joint positions of the first joint assembly and the joint assembly which is located at the distal end of the first joint assembly. The unknown dynamics parameters (such as the gravitational torque $G(\theta)$ in the Formula (2)) can be identified by using the methods such as the least square method by combination the actual torque of the first joint assembly, the friction torque of the first joint assembly, and the joint positions of the first joint assembly and the joint assembly which is located at the distal end of the first joint assembly. For example, in the Formula (2), the identified unknown dynamics parameters mainly include the gravitational torque parameters (including the mass, the barycenter and the like.), thus, the second dynamics model which is associated with the relationship between the joint positions and the first torque can be effectively constructed, in which, the joint positions include the joint position of the first joint assembly and joint position of the joint assembly which is located at the first joint assembly, the first torque include the first torque of the first joint assembly.

In one embodiment, the second torque may be configured for restoring the joint position of the first joint assembly, to perform the effect like a reset spring, and to facilitate the first joint assembly to restore the joint position after the collision. The processor may be configured for:

obtaining an initial joint position and a current joint position of the first joint assembly detected by a sensor; calculating a joint position variable of the first joint assembly based on the initial joint position and the current joint position; and determining the second torque outputted by the actuating device corresponding to the first joint assembly based on the joint position variable. In this case, the initial joint position includes the joint position before the collision.

Preferably, the second torque increases as the joint position variable increases, that is, the larger the joint position variable, the larger the braking force; and the smaller the joint position variable, the smaller the braking force. More preferably, the second torque increases linearly as the joint position variable increases.

For example, the relationship between the second torque and the joint position variable can be expressed as below:

$$\tau = k * \Delta x \qquad \text{Formula (4)},$$

in which, $\tau$ is the second torque, $k$ is a stiffness coefficient, and $\Delta x$ is the joint position variable.

In some embodiments, the second torque may also be configured for braking the motion of the first joint assembly under the collision and therefore may function like a brake, to facilitate braking of the first joint assembly under the collision. The processor may be configured for:

obtaining the current joint position of the first joint assembly detected by the sensor; determining whether the current joint position reaches a first limiting joint position of the first joint assembly, in which, the first limiting joint position is within a range of a physical limiting joint position of the first joint assembly; in response to that the current joint position reaches the first limiting joint position, calculating a joint position variable between the current joint position and the first limiting joint position; and determining the second torque outputted by the actuating device corresponding to the first joint assembly based on the joint position variable.

Preferably, the second torque increases as the joint position variable increases. More preferably, the second torque increases linearly as the joint position variable increases. For example, the Formula (4) can also be used for realizing braking, which is not repeated herein. In this case, the first joint assembly can move freely under the collision in response to that the first joint assembly does not reach the first joint position, and the restoring force for resisting the first joint assembly to move to the physical limiting position to realize braking can be generated from the first joint position in response to that the first joint assembly reaches the first joint position. During the non-braking stage, the first joint assembly can be avoided quickly under the effect force caused by the collision, and during the braking stage, the first joint assembly can be braked progressively by using the spring principle to avoid the unexpected problems caused by the rapid braking.

In one embodiment, when the second torque is the braking torque, the processor may also be configured for:

obtaining an initial joint position and a current joint position of the first joint assembly detected by a sensor; calculating a joint speed of the first joint assembly based on the initial joint position, the current joint position, and a time during which the initial joint position changes to the current joint position; and determining the second torque outputted by the actuating device corresponding to the first joint assembly based on the joint speed.

Preferably, the second torque increases as the joint speed increases, that is, the faster the joint speed, the larger the braking force; and the slower the joint speed, the smaller the braking force. For example, the relationship between the second torque and the joint speed can be expressed as below:

$$\tau = k * v^{n} \qquad \text{Formula (5)},$$

in which, $\tau$ is the second torque, $k$ is a damping coefficient, $v$ is the joint speed, n is an integer greater than or equal to one, for example, n = 1, or n = 2, or n = 3. In this case, when n = 1, the braking effect is more linear, and when n = 2, or n = 3, compared to the case where n = 1, the braking is quicker and more agile.

In the above embodiments, the processor can adjust the output torque of the first joint assembly according to the output resultant torque which is the sum of the determined first torque and the determined second torque, and a good effect associated with the way the second torque acquired can be achieved.

In one embodiment, for example, in the case that the second torque uses a principle of a spring model, that is, using the Formula (4) to realize restoring the joint position of the first joint assembly or braking the motion of the first joint assembly, the processor may also be configured for: recording a holding time of the first joint assembly at the current joint position during a duration of collision, and updating the current joint position to be the initial joint position in response to that the holding time reaches a configured time threshold. In the embodiment, it can be recorded that the holding time of the first joint assembly at the current joint position begins at the time when it is determined that the collision happens. And/or, the processor may also be configured for: obtaining the current joint position of the first joint assembly during the duration of the collision, recording a first holding time in which a variable of the current joint position is lower than a position variation threshold in response to that the variable of the current joint position is lower than the position variation threshold, and updating the current joint position to be the initial joint position in response to that the first holding time reaches a first configured time threshold. For the rotary joint assembly of the first joint assembly, the range of the position variation threshold may include 0 - 0.1°, for example, the position variation threshold may be configured to be 0.01°. For the displacement joint assembly of the first joint assembly, the range of the position variation threshold may include 0 - 0.1 mm, for example, the position variation threshold may be configured to be 0.01 mm. And/or, the processor may also be configured for: obtaining a joint speed of the first joint assembly during the duration of the collision, recording a second holding time in which the joint speed is lower than a speed threshold in response to that the joint speed is lower than the speed threshold, and updating the current joint position to be the initial joint position in response to that the second holding time reaches a second configured time threshold. For the rotary joint assembly of the first joint assembly, the range of the speed threshold may include 0 - 0.1°/s, for example, the speed threshold may be configured to be 0.0001 °/s. For the displacement joint assembly of the first joint assembly, the range of the speed threshold may include 0-0.01 mm/s, for example, the speed threshold may be configured to be 0.0001 mm/s. The configured time threshold, the first configured time threshold, and the second configured time threshold can be exactly the same, partially the same or completely different, for example, all can be configured as 4-8 seconds, for example, all can be configured as 6 seconds. For example, the first joint assemblies may include the whole or the partial of the joint assemblies which are configured to be the first joint assemblies, for example, when all the joint assemblies satisfies any of the above conditions, the current joint position is updated to be the initial joint position. In the embodiment, the initial joint positions are updated and no longer the joint positions before the collision. In such condition, exiting the impedance mode (also called a collision mode) can be realized, and the impedance mode can be re-entered only when it is determined that the first arm and/or the device attached to the distal end of the first arm has a collision again. The impedance mode includes: when it is determined that the collision happens, in response to the collision, adjusting the output torque of the actuating device corresponding to the first joint assembly of the joint assemblies to balance the gravitational torque of the load of the distal end of the first joint assembly, and to restore the joint position of the first joint assembly or brake the first joint assembly. In addition, the duration of the collision includes the entire period from the determination of the happening of the collision to the exiting of the impedance mode. During the duration of the collision, the output torque of the actuating device corresponding to the first joint assembly is required to balance the gravitational torque of the load of the distal end of the corresponding first joint assembly, and to restore the joint position of the first joint assembly or to brake the first joint assembly.

In the embodiment, the joint position variable of the first joint assembly may be re-calculated based on the updated initial joint position and the current position, so that the first torque can change periodically, and the operating device can behave as a state of a staged motion. In this way, the first arm's obstruction to collision can be reduced to some extent, more particularly, when the second arm which is being operated collides with the first arm, as the first arm may keep moving such as shifting and/or rotating under the collision when the holding time of the duration of the collision reaches the second configured time threshold, the influence of reducing the range of motion of the second arm caused by the obstruction of the first arm to the second arm can be reduced or even avoided.

In one embodiment, the processor may also be configured for:

obtaining the joint position of the joint assembly of the first arm detected by the sensor; calculating position parameters of a kinematic model of the first arm according to the joint position and a forward kinematics; determining whether the position parameters of the kinematic model reach boundary parameters of an effective space of the first arm, in which, the effective space is at least a part of a Cartesian Space of the first arm; and at least braking a second joint assembly in response to that the position parameters of the kinematic model reach the boundary parameters of the effective space.

In this case, the second joint assembly comprises a joint assembly of the first joint assembly which reaches the boundary parameters of the effective space. It can be understood that all the first joint assemblies may also be braked in response to that the position parameters of the kinematic model reach the boundary parameters of the effective space. Suitable for different purposes, the effective space can be further restricted or limited. For example, in response to that the distal end of the first arm is mounted with the surgical instrument 150, the effective space of the first arm can be restricted or limited to prevent that the surgical instrument 150 which is being inserted into the body damages the human organs or tissues by following the first arm to shift with large scope under the collision. For example, the initial effective space obtained based on the Cartesian Space of the first arm can be limited according to a certain scale (less than 1) to obtain a new effective space which meets the needs.

In the embodiment, for example, when braking the second joint assemblies only, the first joint assemblies except the second joint assemblies can continue to move under the collision, to better buffer the collision, more particularly, when the second arm being operated is collided with the first arm, the influence of reducing the range of motion of the second arm caused by the obstruction of the first arm to the second arm can be reduced or even avoided. In addition, the implementation method that outputs the second torque for restoring the joint position of the first joint assemblies by the actuating devices of the first joint assemblies during the collision, or the implementation method that outputs the second torque for braking the first joint assemblies by the actuating devices of the first joint assemblies after the collision in the above embodiments can be combined to ensure the reliability and safety in the use of the first arm.

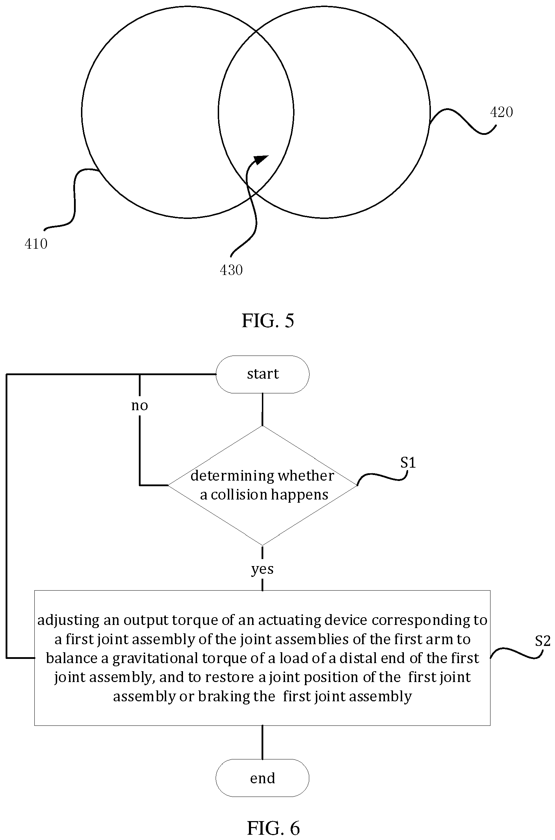

In the embodiment, as illustrated in FIG. 5, in condition that the Cartesian Space 410 of the not being operated first arm and the Cartesian Space 420 of the second arm have an overlapping part 430, the second arm herein refers to the arm (include the sub-arm) which does not collide with the first arm currently, the second arm may be the arm not being operated, or may also be the arm being operated. The effective space of the first arm may be determined based on whether the position parameters of the kinematic model of the second arm are located within the overlapping part 430. For example, when the position parameters of the kinematic model of the second arm are within the overlapping part 430, the effective space of the first arm is the part of the Cartesian Space 410 of the first arm that excludes the overlapping part 430. And/or, when the position parameters of the kinematic model of the second arm are not within the overlapping part 430, the effective space of the first arm is the entire Cartesian Space 410 of the first arm. Such a design can enlarge the range of motion of the first arm under the collision as much as possible. Furthermore, such a design can avoid the collision from being transmitted from the first arm to the second arm, more particularly, when the second arm is the arm being operated, by avoiding the transmission of the collision, and it can be ensured that the second arm is able to smoothly perform the surgical operations without interference.

In some embodiments, the processor may also be configured for: determining whether the condition is satisfied before the collision; and in response to determination that the condition is satisfied, adjusting the output torque of the actuating device corresponding to the first joint assembly of the joint assemblies to balance the gravitational torque of the load of the distal end of the corresponding first joint assembly. And then, when it is determined that the first arm and/or the device attached to the distal end of the first arm collides, in response to the collision of the first arm and/or the device attached to the distal end of the first arm, the output torque of the actuating device corresponding to the first joint assembly is adjusted for balancing the gravitational torque generated by the load of the distal end of the first joint assembly, and the joint position of the first joint assembly is restored or the first joint assembly is braked. According to the embodiment, the first arm can be prepared for the collision before the collision, for example, for the case that even if the force caused by the collision is relatively small, and even cannot be determined as a collision by the system, some buffering effect can also be achieved.

In this embodiment, said determination that the condition is satisfied as mentioned in above may include obtaining a trigger signal.

For example, the surgical robot may also include another input device which is electrically connected to the processor, the another input device may include but not limited to one or more of a button, a switch, a touch screen, a voice recognition device, a fingerprint recognition device, a facial recognition device, and the like, and the trigger signal may be inputted by controlling the another input device by the user.

For example, the surgical robot may also include a sensor which is electrically connected to the processor, the sensor is configured for detecting whether the distal end of the first arm is attached with the device. The trigger signal may be generated automatically by the sensor when it is detected that the distal end of the first arm does not attach with the device, for example, the device may include one or more of a puncture device, and the surgical instrument 150, etc., which are detachably connected to the distal end of the first arm.

In some embodiments, the surgical robot may also include a sensor which is electrically connected to the processor and configured for detecting whether the distal end of the first arm is attached with the device, the processor may be specifically configured for:

In case that it is detected that the distal end of the first arm is attached with the device, in response to a collision of the first arm and/or the device attached to the distal end of the first arm, adjusting the output torque of the actuating device corresponding to the first joint assembly for balancing the gravitational torque generated by the load of the distal end of the first joint assembly, and to restore the joint position of the first joint assembly or brake the first joint assembly. In the embodiment, for example, in the slave operating device as illustrated in FIG. 1, if the distal end of some arm (includes the sub-arm) does not attach with the device, it is indicated that the arm is an idle arm, and during the operations, such arm is generally arranged in a non-surgical area to be away from the other arm which being operated, and therefore will not to be collided by the arm which being operated. And then, some arm which being collided generally is the arm that arranged in a surgical area, and the arm arranged in the surgical area generally should mounted with the device. Therefore, the embodiment allows to execute the above procedures in response to the collision of the first arm and/or the device attached to the distal end of the first arm when it is detected that the distal end of the first arm is attached with the device.

In some embodiments, the surgical robot may also include a sensor which is electrically connected to the processor and configured for detecting the type information of the surgical instrument 150, and may also include the actuating devices which are electrically connected to the processor and configured for actuating the end effector of the distal end of the surgical instrument 150 to work. The processor may be specifically configured for:

when it is determined that the type information of the surgical instrument 150 and/or the state information of the end effector satisfies the safety conditions, in response to the collision of the first arm and/or the device attached to the distal end of the first arm, adjusting the output torque of the actuating device corresponding to the first joint assembly for balancing the gravitational torque generated by the load of the distal end of the first joint assembly, and to restore the joint position of the first joint assembly or brake the first joint assembly.

In the embodiment, for example, when the surgical instrument 150 is an endoscopic, the safety conditions can be considered to be satisfied. For another example, when the surgical instrument 150 is a cautery hook, if the cautery hook is in power on state, the safety conditions can be considered to be satisfied, and if the cautery hook is in power on state, the safety conditions can be considered to be unsatisfied. For example, when the surgical instrument 150 is a scissor, if the scissor is in closed state, the safety conditions can be considered to be satisfied, and if the scissor is in opened state, the safety conditions can be considered to be unsatisfied. Not listed herein. In this way, the buffering of the collision can be realized while ensuring the reliability and safety of the surgical procedure.

In some embodiments, as illustrated in FIG. 1, in the case where the first arm only includes the manipulator 130, or, in the case where the first arm only includes the tornado rotational joint assembly of the adjusting arm 120 and the manipulator 130, even if the distal end of the first arm is mounted with the device as described above, and even if the first arm and/or the device attached to the distal end of the first arm has the collision, when any of the first joint assemblies of the first arm rotates, the manipulator 130 will only move around the remote center of motion, therefore having relatively low risk of potential damage to organs and tissues in the body.

In some embodiments, when the second arm is the arm being operated, for example, in the slave operating device as illustrated in FIG. 1, the second arm includes the manipulator and the surgical instrument, the motion input device manipulates the manipulator and the surgical instrument in a coordinated motion to perform the operations. In some embodiments, when the surgical robot includes the second arm which being operated, the processor can also be configured for: recording a holding time in which the current signals reach a configured current threshold in response to that the current signals of the actuating device of the second arm detected by the sensor of the second arm reaches the configured current threshold; determining whether the holding time reaches a time threshold; and sending a command to the actuating device of the second arm to control the actuating device of the second arm to stop moving in response to that the holding time reaches the time threshold, so as to stop the corresponding joint assembly actuated by the actuating device from moving. In this case, the reason for that the current signals of the actuating device of the second arm reach the configured threshold may be the collision, and may also be the others. According to the embodiment, overcurrent protection for the second arm can be achieved.

In this case, when the current signals of any of the actuating devices of the second arm reach the configured current threshold and the holding time reaches the time threshold, it is required to send the commands to control at least one actuating device of the second arm to stop moving, for example, commands can be sent to control all the actuating devices of the second arm to stop moving.

The commands used for controlling the at least one actuating device of the second arm to stop moving may include a command position and/or a command speed. For example, taking the commands being the command speed as an example, the command speed can be obtained by using the formula as below:

$$V = V_1 \text{-} \Delta V \qquad \text{Formula (6),}$$

in which, V is the command speed, $V_1$ is the current joint speed, $\Delta V$ is the joint speed variable. The joint speed of the joint assembly may be determined by the speed of the actuating of the corresponding actuating device.

The joint speed (with respect to the speed of the actuating device) of the corresponding joint assembly can be reduced to 0 from the current joint speed $V_1$ within the specified cycle(s). For example, trapezoidal velocity planning can be used to achieve a stop motion and thus to determine $\Delta V$. In a simpler example, assuming that the current joint speed of a joint assembly is 10 mm/s, and it need to reduce the current joint speed to 0 from 10 mm/s within 10 cycles, for example, it can define the $\Delta V$ as a fixed value which is 1 mm/s, and the command speed can be sent once per cycle in order to reduce the current joint speed by 1 mm/s during that cycle, for example, the command speed is 9 mm/s during the first cycle, the command speed is 8 mm/s during the second cycle, and the like, until the current joint speed is reduced to 0. It can be understood that $\Delta V$ may also be a variation value, for example, the required $\Delta V$ during each cycle can also be determined by trajectory planning (includes speed planning), so as to determine the command speed, which is not listed herein.

The present disclosure may also provide an anti-collision method for the arm of the surgical robot. As illustrated in FIG. 6, in some embodiments, the method may include:

Step S1, determining whether the first arm and/or the device attached to the distal end of the first arm collides.

In Step S1, when it is determined that the first arm and/or the device attached to the distal end of the first arm collides, Step S1 is performed.

Step S2, in response to the collision of the first arm and/or the device attached to the distal end of the first arm, adjusting an output torque of an actuating device corresponding to a first joint assembly of the joint assemblies of the first arm for balancing a gravitational torque generated by a load of a distal end of the first joint assembly, and to restore a joint position of the first joint assembly or brake the first joint assembly.

The "balance" described in Step S2 generally means a relatively balanced relationship, which allows the presence of the torque deviation. For a same first joint assembly, the balance may be considered to be realized when the following formula is satisfied:

$$x = \left| F_1 - F_2 \right| \le \Delta x$$

$x$ is the torque deviation, F1 is the output torque of the actuating device, F2 is the gravitational torque of the load of the distal end of the corresponding first joint assembly,

$\Delta x$ is the torque deviation threshold. For example, the range of values of $\Delta x$ may be [0, 10] N/m.

In this case, as long as $x \le \Delta x$, the output torque of the actuating device can be considered to balance the gravitational torque of the load of the distal end of the corresponding joint assembly.

And then, when the first arm is collided, the adjusted joint assembly of the first arm, which is the first joint assembly, can be deflected and/or moved easily under the effect of the external force, to effectively buffer the external force by the changes in position and/or in orientation.

In some embodiments, the first joint assembly of the first arm may be formed by a joint assembly which is configured by the system or the user. In the embodiments, which joint assembly of the first arm is exactly included by the first joint assembly is pre-determined (that is, pre-configured) before the collision. For example, the first joint assembly of the first arm can be obtained from the system files, and the first joint assembly of the first arm may also be configured by the user configuration interface. In this case, the first joint assembly generally means a type of joint assembly, and the number of the first joint assembly includes at least one. For example, when the first joint assemblies include at least two joint assemblies, the processor can be configured to adjust the output torque of the actuating device corresponding to each first joint assembly to balance the gravitational torque of the load of the distal end of the corresponding first joint assembly respectively.

For example, in the slave operating device as illustrated in FIG. 1, the manipulator can be configured as the first arm. Optionally, for example, both of the pitch joint assembly and the yaw joint assembly of the manipulator can be configured as the first joint assemblies of the first arm. In this example, if the force caused by the collision has component forces in the pitch DOF corresponding to the pitch joint assembly and/or in the yaw DOF corresponding to the yaw joint assembly, by adjusting the output torque of the actuating device of the pitch joint assembly to be the gravitational torque of the load of the distal end of the pitch joint assembly, at the same time, by adjusting the output torque of the actuating device of the yaw joint assembly to be the gravitational torque of the load of the distal end of the yaw joint assembly, an effective buffering effect for the collision can be achieved.

In some embodiments, the first joint assembly of the first arm may also not be completely configured by the system or the user, but determined based on the position of the crash point located at the first arm and/or the device attached to the distal end of the first arm, which is relatively more flexible, that is, in some extent, the changes in position of the crash point may affect the determination of the first joint assembly. In this case, the joint assembly having a buffering effect for the collision is generally a joint assembly where the crash point is located and/or a joint assembly which is in proximity to the proximal end of the j oint assembly where the crash point is located.

For example, the first joint assembly may include at least one component as below: the joint assembly where the crash point is located, and the joint assembly which is in proximity to the proximal end of the joint assembly where the crash point is located. For example, all of the joint assembly where the crash point is located and the joint assemblies which are in proximity to the proximal end of the joint assembly where the crash point is located may be configured as the first joint assemblies according to the pre-configured configuration strategy. For another example, the joint assembly where the crash point is located may be configured as the first joint assembly according to the pre-configured configuration strategy. For another example, all of the joint assemblies which are in proximity to the proximal end of the joint assembly where the crash point is located may be configured as the first joint assemblies according to the pre-configured configuration strategy.

For example, the first joint assembly may include at least one component as below: the joint assembly where the crash point is located, and the joint assembly which is in proximity to the proximal end of the j oint assembly where the crash point is located. At the same time, the first joint assemblies may further include the joint assemblies which are in proximity to the distal end of the joint assembly where the crash point is located. In this case, as the joint assembly which is in proximity to the distal end of the joint assembly where the crash point is located may have no effective buffering effect for the collision when collision occurs, so that it can be determined whether to configure the joint assembly which is in proximity to the distal end of the joint assembly where the crash point is located to be the first joint assembly.

In this case, the joint assembly where the crash point is located, and the joint assembly which is in proximity to the proximal end of the joint assembly where the crash point is located, and the joint assembly which is in proximity to the distal end of the joint assembly where the crash point is located may respectively include more than one joint assemblies.

The joint assembly where the crash point is located may include two cases. The first case is the crash point is actually located at a certain joint assembly of the first arm. The second case is the crash point is actually not located at any joint assembly of the first arm, but is located at the device attached to a distal end of the first arm, in such case, a joint assembly of the first arm which is in proximity to the device can be configured as the joint assembly where the crash point is located.

In some embodiments, whichever way the first joint assembly is determined, the joint assemblies corresponding to the DOFs of the operating device as many as possible can be configured to be the first joint assemblies. When the first joint assemblies include a plurality of joint assemblies and have no redundant DOF, the collision can be buffered from a multiple directions. When the first joint assemblies include a plurality of joint assemblies and have at least one redundant DOF, a multi-level buffering can be achieved by using the joint assemblies corresponding to the at least one redundant DOF for the collision from the at least one direction corresponding to the at least one redundant DOF.

In some embodiments, the first arm at least includes an active joint assembly, the active joint assembly refers to the joint assembly which is actuated by the actuating device. The first joint assembly generally refers to the active joint assembly.

In some embodiments, the output torque of the actuating device corresponding to the first joint assembly includes an output resultant torque. The output resultant torque includes a first torque and a second torque, for example, the output torque includes a sum of the first torque and the second torque. In one embodiment, the first torque may be configured for balancing the gravitational torque of the load of distal end of the first joint assembly. The second torque may be configured for restoring the joint position of the first joint assembly, or, the second torque may be configured for braking the first joint assembly.

In some embodiments, the "collision" described in the present disclosure may include object-to-object collision and person-to-object collision. Broadly speaking, the "collision" may also include the object receiving the external force. In this case, the "object" mainly refers to the arm in the present disclosure, such as the first arm of the arms.

The present disclosure may be used in a variety of ways, alone or in combination, to determine whether the first arm has a collision.

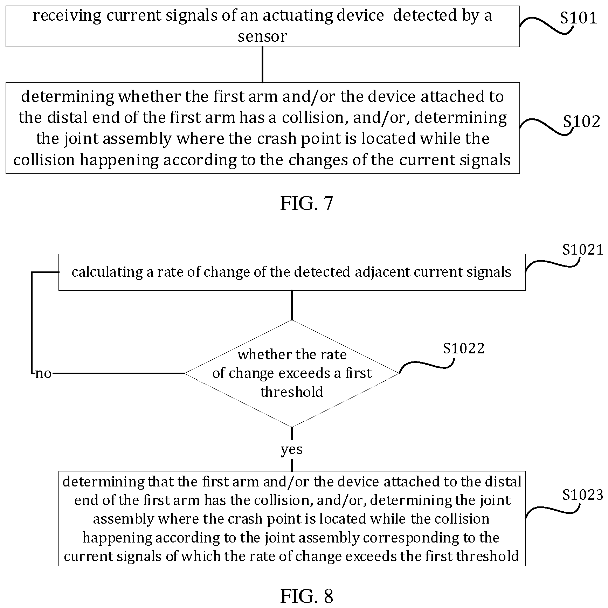

In some embodiments, as illustrated in FIG. 7, the above Step S1, that is, the step of determining whether the first arm and/or the device attached to the distal end of the first arm collides may also include:

Step S101, receiving current signals of an actuating device detected by a sensor.
Step S102, determining whether the first arm and/or the device attached to the distal end of the first arm has a collision, and/or, determining the joint assembly where the crash point is located while the collision happening according to the changes of the current signals.

In the embodiment, the sensors is specifically configured for detecting the current signals at intervals of a first cycle, as illustrated in FIG. 8, the above Step S102 may also include:

Step S1021, calculating a rate of change of the detected adjacent current signals.
Step S1022, determining whether the rate of change exceeds a first threshold.

In Step S1022, when the rate of change exceeds the first threshold, Step S1023 is performed.

Step S1023, determining that the first arm and/or the device attached to the distal end of the first arm has the collision, and/or, determining the joint assembly where the crash point is located while the collision happening according to the joint assembly corresponding to the current signals of which the rate of change exceeds the first threshold.

It can be understood that when the rate of change does not exceed the first threshold, whether the first arm and/or the device attached to the distal end of the first arm has the collision cannot be determined temporarily, and it cannot be determined temporarily the joint assembly where the crash point is located while the collision happening according to the joint assembly corresponding to the current signals of which the rate of change exceeds the first threshold, either.

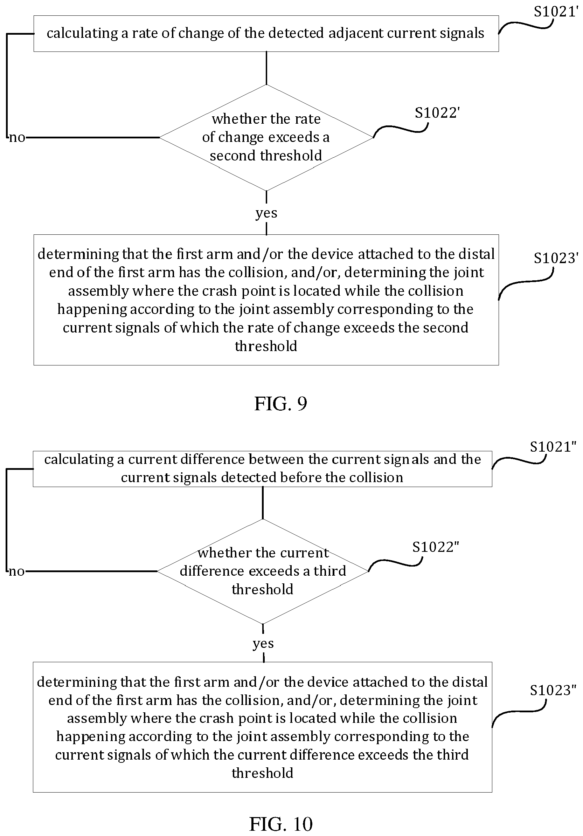

In the embodiment, the sensor is specifically configured for detecting the current signals at intervals of a second cycle, and the second cycle is larger than the first cycle, as illustrated in FIG. 9, the above Step S 102 may also include:

Step S1021', calculating a rate of change of the detected adjacent current signals.
Step S 1022', determining whether the rate of change exceeds a second threshold.

The second threshold is smaller than the first threshold. In Step S 1022', when the rate of change exceeds the second threshold, Step S1023' is performed.

Step S1023', determining that the first arm and/or the device attached to the distal end of the first arm has the collision, and/or, determining the joint assembly where the crash point is located while the collision happening according to the joint assembly corresponding to the current signals of which the rate of change exceeds the second threshold.

It can be understood that when the rate of change does not exceed the second threshold, whether the first arm and/or the device attached to the distal end of the first arm has the collision cannot be determined temporarily, and it cannot be determined temporarily the joint assembly where the crash point is located while the collision happening according to the joint assembly corresponding to the current signals of which the rate of change exceeds the second threshold, either.

For example, assuming the first cycle is 1 ms, in response to that the rate of change corresponding to the first cycle exceeds the first threshold, it can be determined that the first arm and/or the device attached to the distal end of the first arm has the collision, and the joint assembly where the crash point is located while the collision happening can also be determined.

When the rate of change of the current signals is lower, but the variation of the final current signals with the accumulation of time is higher, in fact, it can also be considered that the collision happens. Under this condition, for example, if it is otherwise directly concluded that the first arm and/or the device attached to the distal end of the first arm does not have the collision based on that the rate of change corresponding to the first cycle does not exceed the threshold, then an incorrect determination result may occur, leading to a failure to buffer the collision.

And then, at the same time, the sensor may also detect the current signals at intervals of the second cycle. The second cycle such as 100 ms, in response to that the rate of change corresponding to the second cycle exceeds the second threshold which is smaller than the first threshold, then it can be determined that the first arm and/or the device attached to the distal end of the first arm has the collision, and the joint assembly where the crash point is located while the collision happening can also be determined.

Similar to the principle, the sensor may also detect the current signals at intervals of a third cycle and even more cycle, and the thresholds of the rates of change corresponding to the different cycle are different, in which, the longer the cycle, the smaller the corresponding threshold may be configured to be. In response to that the rate of change corresponding to any cycle exceeds the corresponding threshold, then it can be determined that the first arm and/or the device attached to the distal end of the first arm has the collision, and the joint assembly where the crash point is located while the collision happening can also be determined.

In the embodiment, as illustrated in FIG. 10, the above Step S102 may also include:

Step S1021", calculating a current difference between the current signals and the current signals detected before the collision.

Step S1022", determining whether the current difference exceeds a third threshold.

In Step S1022", when the current difference exceeds the third threshold, Step S1023" is performed.

Step S1023", determining that the first arm and/or the device attached to the distal end of the first arm has the collision, and/or, determining the joint assembly where the crash point is located while the collision happening according to the joint assembly corresponding to the current signals of which the current difference exceeds the third threshold.

It can be understood that in response to that the current difference does not exceed the third threshold, whether the first arm and/or the device attached to the distal end of the first arm has the collision cannot be determined temporarily, and it cannot be determined temporarily the joint assembly where the crash point is located while the collision happening according to the joint assembly corresponding to the current signals of which the current difference exceeds the third threshold, either.

In the embodiment, the way to determine whether the first arm and/or the device attached to the distal end of the first arm has the collision, and/or, determine the joint assembly where the crash point is located while the collision happening according to whether the current difference exceeds the corresponding threshold, can be used in combination with the way to determine whether the first arm and/or the device attached to the distal end of the first arm has the collision, and/or, determine the joint assembly where the crash point is located while the collision happening according to whether the rate of change of the detected current signals exceeds the corresponding threshold, to determine whether the first arm and/or the device attached to the distal end of the first arm has the collision and/or determine the joint assembly where the crash point is located while the collision happening more accurately. In this case, in response to that any of these determination results exceeds the corresponding threshold, it can be determined that the first arm and/or the device attached to the distal end of the first arm has the collision, and the joint assembly where the crash point is located while the collision happening can also be determined. And then, in response to that none of these determination results does not exceed the corresponding threshold, whether the first arm and/or the device attached to the distal end of the first arm has the collision cannot be determined temporarily, and it cannot be determined temporarily the joint assembly where the crash point is located while the collision happening, either.

In fact, the crash point of the first arm may not be limited to only one. Even if the crash points are more than two, the joint assemblies where the crash points located can also be determined according to the above embodiments, and even if there are a plurality of the crash points, an effective buffering effect for the collision can also be achieved according to the above embodiments of the present disclosure.

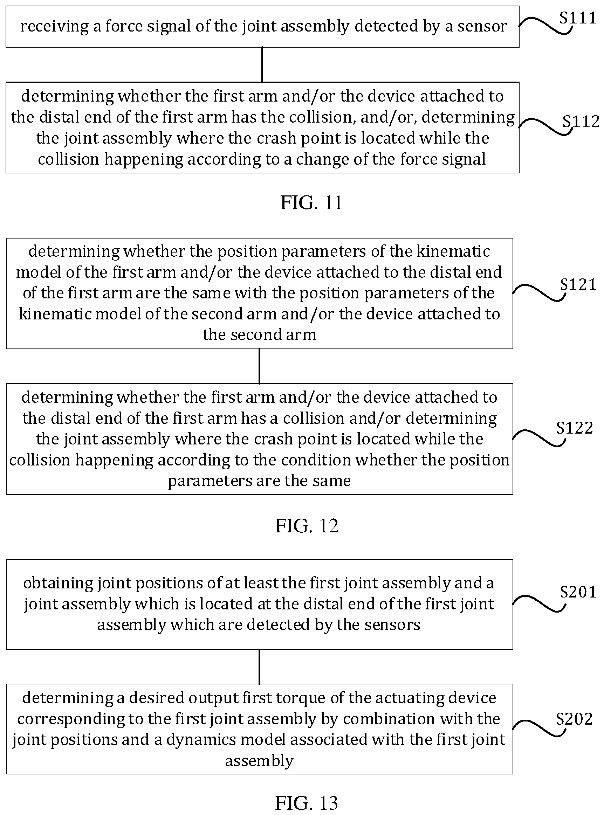

In one embodiment, as illustrated in FIG. 11, the above Step S1, that is, the step of determining whether the first arm and/or the device attached to the distal end of the first arm collides may also include:

Step S111, receiving a force signal of the joint assembly detected by a sensor.
Step S112, determining whether the first arm and/or the device attached to the distal end of the first arm has the collision, and/or, determining the joint assembly where the crash point is located while the collision happening according to a change of the force signal.

In the embodiments, the processor is specifically configured for: determining whether the force signal is higher than a configured threshold; in response to that a detected force signal is higher than the threshold, then determining that the first arm and/or the device attached to the distal end of the first arm has the collision, otherwise, determining the first arm and/or the device attached to the distal end of the first arm does not have the collision, and optionally, determining the joint assembly where the crash point is located while the collision happening. In this case, the joint assembly where the crash point is located while the collision happening may be the joint assembly corresponding to the sensor that the force signals is higher than the threshold.

In the above embodiments, for the case that the first joint assembly is the pre-configured joint assembly, generally, it is not required to determine the joint assembly where the crash point is located while the collision happening, and it is only required to determine whether the first arm and/or the device attached to the distal end of the first arm has the collision. But for the case that the determination of the first joint assembly is associated with the locations of the crash points, it is required to determine whether the first arm and/or the device attached to the distal end of the first arm has the collision, and it is also required to determine the joint assembly where the crash point is located while the collision happening.

In the above embodiments, for the case that the first joint assembly is the pre-configured joint assembly, the processed current signals at least include the current signals of the actuating device corresponding to the first joint assembly, for example, only include the current signals of the actuating device corresponding to the first joint assembly, and/or, the processed force signals at least include the force signals of the first joint assembly, for example, only include the force signals of the first joint assembly.

In another embodiment, generally, the surgical robot may also include a second arm, as illustrated in FIG. 12, the above Step S1, that is, the step of determining whether the first arm and/or the device attached to the distal end of the first arm collides may also include:

Step S121, determining whether the position parameters of the kinematic model of the first arm and/or the device attached to the distal end of the first arm are the same with the position parameters of the kinematic model of the second arm and/or the device attached to the second arm.

Step S122, determining whether the first arm and/or the device attached to the distal end of the first arm has a collision and/or determining the joint assembly where the crash point is located while the collision happening according to the condition whether the position parameters are the same.

The "same" described in Step S122 may include the same and the approximately the same.

Specifically, in response to that the position parameters of the kinematic model of the first arm and/or the device attached to the distal end of the first arm are the same with (that is, coincide with) the position parameters of the kinematic model of the second arm and/or the device attached to the second arm, determining that the first arm and/or the device attached to the distal end of the first arm has the collision, and/or, determining the joint assembly where the crash point is located while the collision happening according to the joint assemblies where the same position parameters located. In response to that the position parameters of the kinematic model of the first arm and/or the device attached to the distal end of the first arm are not the same with the position parameters of the kinematic model of the second arm and/or the device attached to the second arm, determining that the first arm and/or the device attached to the distal end of the first arm does not have the collision temporarily.

In one embodiment, as illustrated in FIG. 13, specifically, in the above Step S2, that is, before the step of adjusting the output torque of the actuating device corresponding to the first joint assembly of the joint assemblies of the first arm to balance the gravitational torque generated by the load of the distal end of the first joint assembly, and to restore the joint position of the first joint assembly or brake the first joint assembly, the method may also include:

Step S201, obtaining joint positions of at least the first joint assembly and a joint assembly which is located at the distal end of the first joint assembly which are detected by the sensors.
Step S202, determining a desired output first torque of the actuating device corresponding to the first joint assembly by combination with the joint positions and a dynamics model associated with the first joint assembly.

The dynamics model required to be used in Step S202 is generally constructed for the first joint assembly. For example, for the different first joint assemblies, the constructed dynamics models are different. Generally, the dynamics model corresponding to the first joint assembly is associated with the first joint assembly and the joint assembly located at the distal end of the first joint assembly of the first arm. For example, for the first arm is the manipulator, when the pitch joint assembly and the yaw joint assembly of the manipulator are configured as the first joint assemblies, then the pitch joint assembly is corresponding to one dynamics model, and the yaw joint assembly is corresponding to another dynamics model. Assuming the yaw joint assembly is located at the proximal end of the pitch joint assembly, then the dynamics model of the pitch joint assembly is only associated with the pitch joint assembly and the joint assembly which is located at the distal end of the pitch joint assembly, and the dynamics model of the yaw joint assembly includes the yaw joint assembly and all the joint assemblies which are associated with the dynamics model of the pitch joint assembly. Understandable, for the case that the first arm is consisted by more than two sub-arms, the corresponding dynamics model is also constructed based on the first joint assembly, which would not be repeated herein.

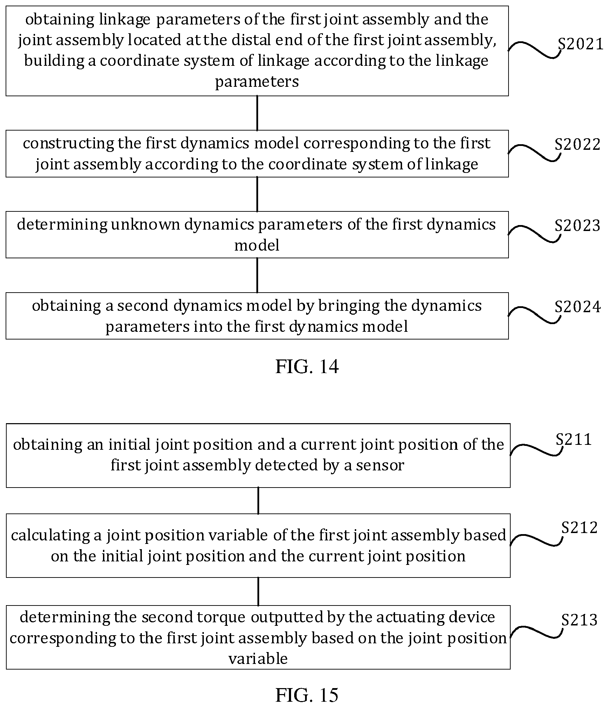

In one embodiment, as illustrated in FIG. 14, before the above Step S202, the dynamics model corresponding to the first joint assembly can be constructed as the following steps, the method may include:

Step S2021, obtaining linkage parameters of the first joint assembly and the joint assembly located at the distal end of the first joint assembly, building a coordinate system of linkage according to the linkage parameters.

The joint assembly includes a joint and a linkage connected to the joint, the linkage parameters (that is, DH parameters) may include parameters, such as, at least one of the joint angle and the joint displacement, the linkage length, and the like.

Step S2022, constructing the first dynamics model corresponding to the first joint assembly according to the coordinate system of linkage.

In this case, the first dynamics model is generally in a symbolic form (that is, a formula with unknown parameters), the first dynamics model is a fuzzy (that is, the parameters of the dynamics are undetermined temporarily) dynamics model. For example, the first dynamics model may be expressed as the following formula:

$$\tau = M(\theta)\ddot{\theta} + H(\theta,\dot{\theta}) + G(\theta) \qquad \text{Formula (1),}$$

in which, $\tau$ is an actual torque of the joint assembly, $\theta$ is the joint position of the joint assembly, $\dot{\theta}$ is a speed of the joint assembly ($\dot{\theta}$ is the first derivative of $\theta$), $\ddot{\theta}$ is the speed of the joint assembly ($\ddot{\theta}$ is the second derivative of $\theta$), $M(\theta)$ is an inertia matrix, $H(\theta,\dot{\theta})$ includes a Koch force and a centrifugal force, and $G(\theta)$ is the gravitational torque of the joint assembly.

Step S2023, determining unknown dynamics parameters of the first dynamics model.

In this case, the first dynamics model generally includes at least one unknown dynamics parameters, the unknown dynamics parameters associated with the formula (1) can be determined respectively, to obtain the exactly second dynamics model. In one embodiment, the contribution for the joint torque by the parts of the unknown dynamics parameters can be ignored according to the actual situation, for example, a few of the key dynamics parameters such as the mass of the joint assembly, the barycenter of the joint assembly and the friction torque of the joint assembly can be mainly focused. In some embodiments, the mass, the barycenter and the friction torque of the joint assembly may be affected by the actuating device which actuating the joint assembly and/or the transmission device which is connected between the actuating device and the joint assembly for transmitting. For example, when the structure of the operating device is relatively regular, at least parts of the dynamics parameters, such as the mass, the barycenter and the friction torque of the joint assembly can be obtained directly without identification. It can be understood that at least parts of the dynamics parameters, such as the mass, barycenter and the friction torque of the joint assembly can also be obtained by identifying. For example, the mass of the joint assembly can be obtained by weighing, the barycenter and the friction torque of the joint assembly can be obtained by identifying. For example, in some embodiments, the contribution for the joint torque by $M(\theta)$ and $H(\theta,\dot{\theta})$ can be ignored in some embodiments of the present disclosure, so that, the Formula (1) can be simplified as:

$$\tau = G(\theta) \qquad \text{Formula (2).}$$

Step S2024, obtaining a second dynamics model by bringing the dynamics parameters into the first dynamics model.

In this case, the second dynamics model is a clear (that is, the dynamics parameters are known) dynamics model. And then, when combining the joint position and the dynamics model corresponding to the first joint assembly to determine a desired output first torque of the actuating device corresponding to the first joint assembly, the used dynamics model is the second dynamics model.

In one embodiment, considering the adverse effects of the friction torque, the friction torque can be excluded from the actual torque of the joint assembly, specifically:

The torque equilibrium model can be constructed based on a dynamic equilibrium principle. The torque equilibrium model may be expressed as the following formula:

$$\tau = k_1 * \sin\theta + k_2 * \cos\theta + f * sign(\dot{\theta}) \qquad \text{Formula (3),}$$

in which, $\tau$ is the actual torque of the joint assembly, $\theta$ is the joint position of the joint assembly, $\dot{\theta}$ is the speed of the joint assembly, $k_1$ and $k_2$ are the gravitational torque parameters, $f$ is the friction torque of the joint assembly, and $sign(\dot{\theta})$ is the direction of the speed.

The friction torque of the joint assembly can be determined by identifying. For example, the individual joint assembly can be controlled to perform a uniform motion at a low speed, and to traverse the entire range of motion, and to capture the actual torque and the corresponding joint position of the joint assembly. The individual joint assembly is the joint assembly corresponding to the first joint assembly. In this case, during the procedure of the uniform motion, the friction torque is approximately invariant and is considered to be a constant value, and then, the friction torque of the joint assembly can be identified by using the methods such as the least square method according to the captured actual torque and the corresponding joint position of the joint assembly. Understandably, the actual torque of the joint assembly is outputted by the actuating device which actuates the joint assembly.

When determining the unknown dynamics parameters (such as the gravitational torque $G(\theta)$ in the Formula (2)) in the first dynamics model by identifying, each of the joint assemblies can be controlled to perform a uniform motion at a low speed, and to traverse the entire range of motion, and to capture the actual torque of the first joint assembly, and to capture the corresponding joint positions of the first joint assembly and the joint assembly which is located at the distal end of the first joint assembly. The unknown dynamics parameters (such as the gravitational torque $G(\theta)$ in the Formula (2)) can be identified by using the methods such as the least square method by combination the actual torque of the first joint assembly, the friction torque of the first joint assembly, and the joint positions of the first joint assembly and the joint assembly which is located at the distal end of the first joint assembly. For example, in the Formula (2), the identified unknown dynamics parameters mainly include the gravitational torque parameters (including the mass, the barycenter and the like.), thus, the second dynamics model which is associated with the relationship between the joint positions and the first torque can be effectively constructed, in which, the joint positions include the joint position of the first joint assembly and joint position of the joint assembly which is located at the first joint assembly, the first torque include the first torque of the first joint assembly.

In one embodiment, the second torque may be configured for restoring the joint position of the first joint assembly, to perform the effect like a reset spring, and to facilitate the first joint assembly to restore the joint position after the collision. As illustrated in FIG. 15, specifically, in the above Step S2, that is, before the step of adjusting the output torque of the actuating device corresponding to the first joint assembly of the joint assemblies of the first arm to balance the gravitational torque generated by the load of the distal end of the first joint assembly, and to restore the joint position of the first joint assembly or brake the first joint assembly, the method may also include:

Step S211, obtaining an initial joint position and a current joint position of the first joint assembly detected by a sensor.

The initial joint position includes the joint position before the collision.

Step S212, calculating a joint position variable of the first joint assembly based on the initial joint position and the current joint position.

Step S213, determining the second torque outputted by the actuating device corresponding to the first joint assembly based on the joint position variable.

Preferably, the second torque increases as the joint position variable increases, that is, the larger the joint position variable, the larger the braking force; and the smaller the joint position variable, the smaller the braking force. More preferably, the second torque increases linearly as the joint position variable increases.

For example, the relationship between the second torque and the joint position variable can be expressed as below:

$$\tau = k * \Delta x \qquad \text{Formula (4),}$$

in which, $\tau$ is the second torque, $k$ is a stiffness coefficient, and $\Delta x$ is the joint position variable.

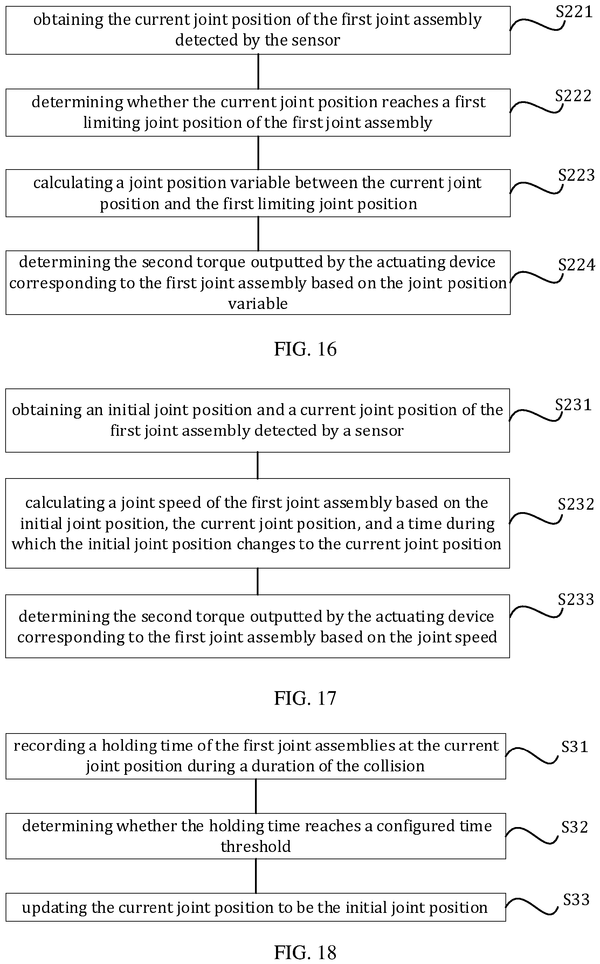

In some embodiments, the second torque may also be configured for braking the motion of the first joint assembly under the collision and therefore may function like a brake, to facilitate braking of the first joint assembly under the collision. As illustrated in FIG. 16, specifically, in the above Step S2, that is, before the step of adjusting the output torque of the actuating device corresponding to the first joint assembly of the joint assemblies of the first arm to balance the gravitational torque generated by the load of the distal end of the first joint assembly, and to restore the joint position of the first joint assembly or brake the first joint assembly, the method may also include:

Step S221, obtaining the current joint position of the first joint assembly detected by the sensor.
Step S222, determining whether the current joint position reaches a first limiting joint position of the first joint assembly.

The first limiting joint position is within a range of the physical limiting joint position of the first joint assembly. In Step S222, in response to that the current joint position reaches the first limiting joint position, Step S223 is performed.

Step S223, calculating a joint position variable between the current joint position and the first limiting joint position.
Step S224, determining the second torque outputted by the actuating device corresponding to the first joint assembly based on the joint position variable.

Preferably, the second torque increases as the joint position variable increases.
More preferably, the second torque increases as the joint position variable increases. More preferably, the second torque increases linearly as the joint position variable increases. For example, the Formula (4) can also be used for realizing braking, which is not repeated herein. In this case, the first joint assembly can move freely under the collision in response to that the first joint assembly does not reach the first joint position, and the restoring force for resisting the first joint assembly to move to the physical limiting position to realize braking can be generated from the first joint position in response to that the first joint assembly reaches the first joint position. During the non-braking stage, the first joint assembly can be avoided quickly under the effect force caused by the collision, and during the braking stage, the first joint assembly can be braked progressively by using the spring principle to avoid the unexpected problems caused by the rapid braking.
In some embodiments, when the second torque is the braking torque, as illustrated in FIG. 17, specifically, in the above Step S2, that is, before the step of adjusting the output torque of the actuating device corresponding to the first joint assembly of the joint assemblies of the first arm to balance the gravitational torque generated by the load of the distal end of the first joint assembly, and to restore the joint position of the first joint assembly or brake the first joint assembly, the method may also include:

Step S231, obtaining an initial joint position and a current joint position of the first joint assembly detected by a sensor.
Step S232, calculating a joint speed of the first joint assembly based on the initial joint position, the current joint position, and a time during which the initial joint position changes to the current joint position.
Step S233, determining the second torque outputted by the actuating device corresponding to the first joint assembly based on the joint speed.

Preferably, the second torque increases as the joint speed increases, that is, the faster the joint speed, the larger the braking force; and the slower the joint speed, the smaller the braking force.
For example, the relationship between the second torque and the joint speed can be expressed as below:

$$\tau = k * v^{n} \qquad \text{Formula (5),}$$

in which, $\tau$ is the second torque, $k$ is a damping coefficient, $v$ is the joint speed, n is an integer greater than or equal to one, for example, n = 1, or n = 2, or n = 3. In this case, when n = 1, the braking effect is more linear, and when n = 2, or n = 3, compared to the case where n = 1, the braking is quicker and more agile.
In the above embodiments, the processor can adjust the output torque of the first joint assembly according to the output resultant torque which is the sum of the determined first torque and the determined second torque, and a good effect associated with the way the second torque acquired can be achieved.
In one embodiment, for example, in the case that the second torque uses a principle of a spring model, that is, using the Formula (4) to realize restoring the joint position of the first joint assembly or braking the motion of the first joint assembly, as shown FIG. 18, the method may also include:
Step S31, recording a holding time of the first joint assemblies at the current joint position during a duration of the collision.
In Step S31, it can be recorded that the holding time of the first joint assembly at the current joint position begins at the time when it is determined that the collision happens.
Step S32, determining whether the holding time reaches a configured time threshold.

In Step S32, in response to that the holding time reaches the configured time threshold, Step S33 is performed.

Step S33, updating the current joint position to be the initial joint position.

In Step S33, the initial joint position is updated and no longer the joint position before the collision. In one embodiment, the above Step S31 - S33 can be combined or replaced as follows: obtaining the current joint position of the first joint assembly during the duration of the collision, recording a first holding time in which a variable of the current joint position is lower than a position variation threshold in response to that the variable of the current joint position is lower than the position variation threshold, and updating the current joint position to be the initial joint position in response to that the first holding time reaches a first configured time threshold. For the rotary joint assembly of the first joint assembly, the range of the position variation threshold may include 0 - 0.1°, for example, the position variation threshold may be configured to be 0.01°. For the displacement joint assembly of the first joint assembly, the range of the position variation threshold may include 0 - 0.1 mm, for example, the position variation threshold may be configured to be 0.01 mm. In one embodiment, the above Step S31 - S33 can be combined or replaced as follows: obtaining a joint speed of the first joint assembly during the duration of the collision, recording a second holding time in which the joint speed is lower than a speed threshold in response to that the joint speed is lower than the speed threshold, and updating the current joint position to be the initial joint position in response to that the second holding time reaches a second configured time threshold. For the rotary joint assembly of the first joint assembly, the range of the speed threshold may include 0 - 0.1°/s, for example, the speed threshold may be configured to be 0.0001°/s. For the displacement joint assembly of the first joint assembly, the range of the speed threshold may include 0-0.01 mm/s, for example, the speed threshold may be configured to be 0.0001 mm/s. The configured time threshold, the first configured time threshold, and the second configured time threshold can be exactly the same, partially the same or completely different, for example, all can be configured as 4-8 seconds, for example, all can be configured as 6 seconds. For example, the first joint assemblies may include the whole or the partial of the joint assemblies which are configured to be the first joint assemblies, for example, when all the joint assemblies satisfies any of the above conditions, the current joint position is updated to be the initial joint position. In such condition, exiting the impedance mode can be realized, and the impedance mode can be re-entered only when it is determined that the first arm and/or the device attached to the distal end of the first arm has a collision again. The impedance mode includes: when it is determined that the collision happens, in response to the collision, adjusting the output torque of the actuating device corresponding to the first joint assembly of the joint assemblies to balance the gravitational torque of the load of the distal end of the first joint assembly, and to restore the joint position of the first joint assembly or brake the first joint assembly. In addition, the duration of the collision includes the entire period from the determination of the happening of the collision to the exiting of the impedance mode. During the duration of the collision, the output torque of the actuating device corresponding to the first joint assembly is required to balance the gravitational torque of the load of the distal end of the corresponding first joint assembly, and to restore the joint position of the first joint assembly or to brake the first joint assembly.

In the embodiment, the joint position variable of the first joint assembly may be re-calculated based on the updated initial joint position and the current position, so that the first torque can change periodically, and the operating device can behave as a state of a staged motion. In this way, the first arm's obstruction to collision can be reduced to some extent, more particularly, when the second arm which is being operated collides with the first arm, as the first arm may keep moving such as shifting and/or rotating under the collision when the holding time of the duration of the collision reaches the second configured time threshold, the influence of reducing the range of motion of the second arm caused by the obstruction of the first arm to the second arm can be reduced or even avoided.

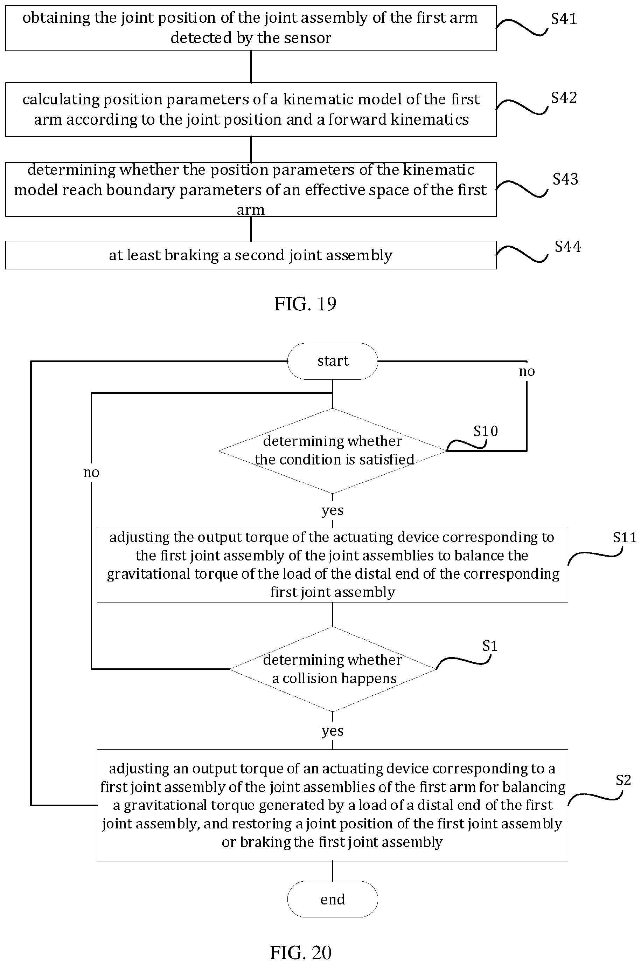

In one embodiment, as illustrated in FIG. 19, the method may also include:

Step S41, obtaining the joint position of the joint assembly of the first arm detected by the sensor.

Step S42, calculating position parameters of a kinematic model of the first arm according to the joint position and a forward kinematics.

Step S43, determining whether the position parameters of the kinematic model reach boundary parameters of an effective space of the first arm.

The effective space is at least a part of a Cartesian Space of the first arm. In Step S43, in response to that the position parameters of the kinematic model reach the boundary parameters of the effective space, Step S44 is performed.

Step S44, at least braking a second joint assembly.

In this case, the second joint assembly comprises a joint assembly of the first joint assembly which reaches the boundary parameters of the effective space. It can be understood that all the first joint assemblies may also be braked in response to that the position parameters of the kinematic model reach the boundary parameters of the effective space. Suitable for different purposes, the effective space can be further restricted or limited. For example, in response to that the distal end of the first arm is mounted with the surgical instrument, the effective space of the first arm can be restricted or limited to prevent that the surgical instrument which is being inserted into the body damages the human organs or tissues by following the first arm to shift with large scope under the collision. For example, the initial effective space obtained based on the Cartesian Space of the first arm can be limited according to a certain scale (less than 1) to obtain a new effective space which meets the needs.

In the embodiment, for example, when braking the second joint assemblies only, the first joint assemblies except the second joint assemblies can continue to move under the collision, to better buffer the collision, more particularly, when the second arm being operated is collided with the first arm, the influence of reducing the range of motion of the second arm caused by the obstruction of the first arm to the second arm can be reduced or even avoided. In addition, the implementation method that outputs the second torque for restoring the joint position of the first joint assemblies by the actuating devices of the first joint assemblies during the collision, or the implementation method that outputs the second torque for braking the first joint assemblies by the actuating devices of the first joint assemblies after the collision in the above embodiments can be combined to ensure the reliability and safety in the use of the first arm.

In the embodiment, as illustrated in FIG. 5, in condition that the Cartesian Space 410 of the not being operated first arm and the Cartesian Space 420 of the second arm have an overlapping part 430, the second arm herein refers to the arm (include the sub-arm) which does not collide with the first arm currently, the second arm may be the arm not being operated, or may also be the arm being operated. The effective space of the first arm may be determined based on whether the position parameters of the kinematic model of the second arm are located within the overlapping part 430. For example, when the position parameters of the kinematic model of the second arm are within the overlapping part 430, the effective space of the first arm is the part of the Cartesian Space 410 of the first arm that excludes the overlapping part 430. And/or, when the position parameters of the kinematic model of the second arm are not within the overlapping part 430, the effective space of the first arm is the entire Cartesian Space 410 of the first arm. Such a design can enlarge the range of motion of the first arm under the collision as much as possible. Furthermore, such a design can avoid the collision from being transmitted from the first arm to the second arm, more particularly, when the second arm is the arm being operated, by avoiding the transmission of the collision, and it can be ensured that the second arm is able to smoothly perform the surgical operations without interference.

In one embodiment, as illustrated in FIG. 20, the method may also include:

Step S10, determining whether the condition is satisfied.

The Step S10 can be executed before the above Step S2, specifically, that is, executed before the step of adjusting the output torque of the actuating device corresponding to the first joint assembly of the joint assemblies of the first arm to balance the gravitational torque generated by the load of the distal end of the first joint assembly, and to restore the joint position of the first joint assembly or brake the first joint assembly. That is, Step S10 may be executed before the collision.

In this case, in Step S10, in response to determination that the condition is satisfied, Step S11 is performed.

Step S11, adjusting the output torque of the actuating device corresponding to the first joint assembly of the joint assemblies to balance the gravitational torque of the load of the distal end of the corresponding first joint assembly.

And then, when it is determined that the first arm and/or the device attached to the distal end of the first arm collides, in response to the collision of the first arm and/or the device attached to the distal end of the first arm, the output torque of the actuating device corresponding to the first joint assembly is adjusted for balancing the gravitational torque generated by the load of the distal end of the first joint assembly, and the joint position of the first joint assembly is restored or the first joint assembly is braked. According to the embodiment, the first arm can be prepared for the collision before the collision, for example, for the case that even if the force caused by the collision is relatively small, and even cannot be determined as a collision by the system, some buffering effect can also be achieved.

In this embodiment, said determination that the condition is satisfied as mentioned in above may include obtaining a trigger signal.

For example, the surgical robot may also include another input device which is electrically connected to the processor, the another input device may include but not limited to one or more of a button, a switch, a touch screen, a voice recognition device, a fingerprint recognition device, a facial recognition device, and the like, and the trigger signal may be inputted by controlling the another input device by the user.

For example, the surgical robot may also include a sensor which is electrically connected to the processor, the sensor is configured for detecting whether the distal end of the first arm is attached with the device. The trigger signal may be generated automatically by the sensor when it is detected that the distal end of the first arm does not attach with the device, for example, the device may include one or more of a puncture device, and the surgical instrument, etc., which are detachably connected to the distal end of the first arm.

In some embodiments, the surgical robot may also include a sensor which is electrically connected to the processor and configured for detecting whether the distal end of the first arm is attached with the device, the processor may be specifically configured for:

in case that it is detected that the distal end of the first arm is attached with the device, in response to a collision of the first arm and/or the device attached to the distal end of the first arm, adjusting the output torque of the actuating device corresponding to the first joint assembly for balancing the gravitational torque generated by the load of the distal end of the first joint assembly, and to restore the joint position of the first joint assembly or brake the first joint assembly. In the embodiment, for example, in the slave operating device as illustrated in FIG. 1, if the distal end of some arm (or a sub-arm) does not attach with the device, it is indicated that the arm (including a sub-arm) is an idle arm, and during the operations, such arm is generally arranged in a non-surgical area to be away from the other arm which being operated, and therefore will not to be collided by the arm which being operated. And then, some arm which being collided generally is the arm that arranged in a surgical area, and the arm arranged in the surgical area generally should mounted with the device. Therefore, the embodiment allows to execute the above procedures in response to the collision of the first arm and/or the device attached to the distal end of the first arm when it is detected that the distal end of the first arm is attached with the device.

In some embodiments, the surgical robot may also include a sensor which is electrically connected to the processor and configured for detecting the type information of the surgical instrument, and may also include the actuating devices which are electrically connected to the processor and configured for actuating the end effector of the distal end of the surgical instrument to work. The processor may be specifically configured for:

when it is determined that the type information of the surgical instrument and/or the state information of the end effector satisfies the safety conditions, in response to the collision of the first arm and/or the device attached to the distal end of the first arm, adjusting the output torque of the actuating device corresponding to the first joint assembly for balancing the gravitational torque generated by the load of the distal end of the first joint assembly, and to restore the joint position of the first joint assembly or brake the first joint assembly.

In the embodiment, for example, when the surgical instrument is an endoscopic, the safety conditions can be considered to be satisfied. For another example, when the surgical instrument is a cautery hook, if the cautery hook is in power on state, the safety conditions can be considered to be satisfied, and if the cautery hook is in power on state, the safety conditions can be considered to be unsatisfied. For example, when the surgical instrument is a scissor, if the scissor is in closed state, the safety conditions can be considered to be satisfied, and if the scissor is in opened state, the safety conditions can be considered to be unsatisfied. Not listed herein. In this way, the buffering of the collision can be realized while ensuring the reliability and safety of the surgical procedure.

In some embodiments, as illustrated in FIG. 1, in the case where the first arm only includes the manipulator, or, in the case where the first arm only includes the tornado rotational joint assembly of the adjusting arm and the manipulator, even if the distal end of the first arm is mounted with the device as described above, and even if the first arm and/or the device attached to the distal end of the first arm has the collision, when any of the first joint assemblies of the first arm rotates, the manipulator will only move around the remote center of motion, therefore having relatively low risk of potential damage to organs and tissues in the body.

In some embodiments, when the second arm is the arm being operated, for example, in the slave operating device as illustrated in FIG. 1, the second arm includes the manipulator and the surgical instrument, the motion input device manipulates the manipulator and the surgical instrument in a coordinated motion to perform the operations. In some embodiments, when the surgical robot includes the second arm which being operated, the processor can also be configured for: recording a holding time in which the current signals reach a configured current threshold in response to that the current signals of the actuating device of the second arm detected by the sensor of the second arm reaches the configured current threshold; determining whether the holding time reaches a time threshold; and sending a command to the actuating device of the second arm to control the actuating device of the second arm to stop moving in response to that the holding time reaches the time threshold, so as to stop the corresponding joint assembly actuated by the actuating device from moving. In this case, the reason for that the current signals of the actuating device of the second arm reach the configured threshold may be the collision, and may also be the others. According to the embodiment, overcurrent protection for the second arm can be achieved.

In this case, when the current signals of any of the actuating devices of the second arm reach the configured current threshold and the holding time reaches the time threshold, it is required to send the commands to control at least one actuating device of the second arm to stop moving, for example, commands can be sent to control all the actuating devices of the second arm to stop moving.

The commands used for controlling the at least one actuating device of the second arm to stop moving may include a command position and/or a command speed. For example, taking the commands being the command speed as an example, the command speed can be obtained by using the formula as below:

$$V = V_1 - \Delta V \qquad \text{Formula (6)},$$

in which, V is the command speed, $V_1$ is the current joint speed, $\Delta V$ is the joint speed variable. The joint speed of the joint assembly may be determined by the speed of the actuating of the corresponding actuating device.

The joint speed (with respect to the speed of the actuating device) of the corresponding joint assembly can be reduced to 0 from the current joint speed $V_1$ within the specified cycle(s). For example, trapezoidal velocity planning can be used to achieve a stop motion and thus to determine $\Delta V$. In a simpler example, assuming that the current joint speed of a joint assembly is 10 mm/s, and it need to reduce the current joint speed to 0 from 10 mm/s within 10 cycles, for example, it can define the ΔV as a fixed value which is 1 mm/s, and the command speed can be sent once per cycle in order to reduce the current joint speed by 1 mm/s during that cycle, for example, the command speed is 9 mm/s during the first cycle, the command speed is 8 mm/s during the second cycle, and the like, until the current joint speed is reduced to 0. It can be understood that ΔV may also be a variation value, for example, the required ΔV during each cycle can also be determined by trajectory planning (includes speed planning), so as to determine the command speed, which is not listed herein.

The present disclosure may also provide a computer-readable storage medium, the computer-readable storage medium stores a computer program, and the computer program is configured to be loaded and executed by a processer to implement the following steps:

adjusting, in response to an external force received by the first arm, an output torque of the actuating device corresponding to a first joint assembly of a joint assembly of the first arm to balance a gravitational torque of a load of a distal end of the first joint assembly, and to restore a joint position of the first joint assembly or brake the first joint assembly.

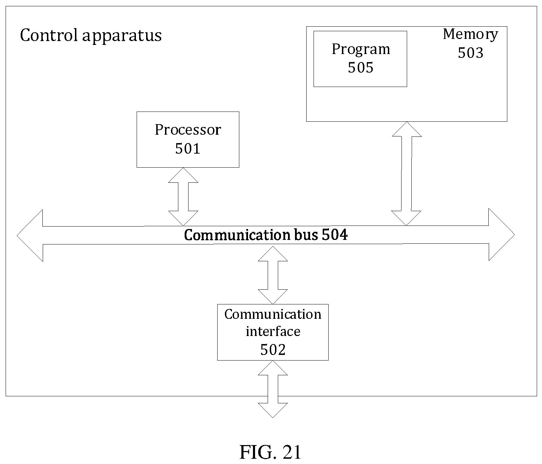

The present disclosure may also provide a system of the surgical robot. As illustrated in FIG. 21, the system may include: a processor 501, a communication interface 502, a memory 503, and a communication bus 504.

The processor 501, the communication interface 502, and the memory 503 can communicate with each other through the communication bus 504.

The communication interface 502 is configured to communicate with network elements of other devices such as various sensors or motors or solenoid valves or other clients or servers, etc.

The processor 501 is configured to execute the program 505, and may specifically execute the corresponding steps in the embodiments of the method mentioned above.

Specifically, the program 505 may include program codes that include computer operation instructions.

The processor 505 may be a central processing unit (CPU), or an ASIC (Application Specific Integrated Circuit), or one or more integrated circuits configured to implement the embodiments of the present disclosure, or a GPU (Graphics Processing Unit). The one or more processors of the control apparatus may be the same type of processors, such as one or more CPU, or one or more GPU. The one or more processors of the control apparatus also may be different types of processors, such as one or more CPU, and one or more GPU.

The memory 503 is configured to store the program 505. The memory 503 may include a high-speed RAM memory, or may further include a non-volatile memory, such as at least one magnetic disk memory.

The program 505 may be specifically configured to make the processor 501 to execute the following operations:

adjusting, in response to an external force received by the first arm, an output torque of the actuating device corresponding to a first joint assembly of a joint assembly of the first arm to balance a gravitational torque of a load of a distal end of the first joint assembly, and to restore a joint position of the first joint assembly or brake the first joint assembly.

The various technical features of the above-described embodiments may be combined in any combination. To make the description concise, not all possible combinations of the various technical features are described in the above-described embodiments. However, as long as the combination of these technical features does not conflict, it should be considered as falling into the scope of the present specification.

The above-described embodiments have only expressed several embodiments of the present application, which are described in detail and specifically, but are not therefore to be construed as limiting the scope of the present application. It should be noted that variations and modifications may be made to one of ordinary skill in the art without departing from the spirit of the present application, all of which fall within the scope of the present application. Therefore, the scope of the application should be subject to the accompanying claims.

**Claims**

1. A surgical robot, **characterized by** comprising:

   at least one arm, the at least one arm comprising a joint assembly and an actuating device for actuating the joint assembly, the at least one arm comprising a first arm; and
   at least one processor, wherein the at least one processor is electrically connected to the actuating device, and the at least one processor is configured for:
   adjusting, in response to an external force received by the first arm, an output torque of the actuating device corresponding to a first joint assembly of the joint assembly of the first arm to balance a gravitational torque of a load of a distal end of the first joint assembly, and to restore a joint position of the first joint assembly or brake the first joint assembly.

**2.** The surgical robot according to claim 1, wherein, the output torque comprises an output resultant torque, the output resultant torque comprises a first torque and a second torque, the first torque is configured for balancing the gravitational torque of the load of the distal end of the first joint assembly, the second torque is configured for restoring the joint position of the first joint assembly or braking the first joint assembly, and the output resultant torque comprises a sum of the first torque and the second torque.

**3.** The surgical robot according to claim 2, wherein, the first arm further comprises a sensor for detecting a joint position of the joint assembly, the at least one processor is electrically connected to the sensor, and the at least one processor is configured for:
obtaining joint positions of at least the first joint assembly and a joint assembly at the distal end of the first joint assembly, and determining a desired output first torque of an actuating device corresponding to the first joint assembly by combination with the joint positions and a dynamics model associated with the first joint assembly.

**4.** The surgical robot according to claim 2, wherein, the first arm further comprises a sensor for detecting a joint position of the joint assembly, the at least one processor is electrically connected to the sensor, and when the second torque comprises a torque for restoring the joint position of the first joint assembly, the at least one processor is configured for:

obtaining an initial joint position and a current joint position of the first joint assembly detected by the sensor;
calculating a joint position variable of the first joint assembly based on the initial joint position and the current joint position; and
determining the second torque outputted by an actuating device corresponding to the first joint assembly based on the joint position variable.

**5.** The surgical robot according to claim 4, wherein, the second torque increases as the joint position variable increases.

**6.** The surgical robot according to claim 5, wherein, a relationship between the second torque and the joint position variable is expressed as a formula:

$$\tau = k * \Delta x,$$

wherein, $\tau$ is the second torque, $k$ is a stiffness coefficient, and $\Delta x$ is the joint position variable.

**7.** The surgical robot according to claim 4, wherein, the at least one processor is further configured for:

recording a holding time of the first joint assembly at the current joint position during a duration of the first arm receiving the external force, and updating the current joint position to be the initial joint position in response to that the holding time reaches a configured time threshold; and/or,
obtaining the current joint position of the first joint assembly during the duration of the first arm receiving the external force, recording a first holding time in which a variable of the current joint position is lower than a position variation threshold in response to that the variable of the current joint position is lower than the position variation threshold, and updating the current joint position to be the initial joint position in response to that the first holding time reaches a first configured time threshold; and/or,

obtaining a joint speed of the first joint assembly during a duration of the first arm receiving the external force, recording a second holding time in which the joint speed is lower than a speed threshold in response to that the joint speed is lower than the speed threshold, and updating the current joint position to be the initial joint position in response to that the second holding time reaches a second configured time threshold.

**8.** The surgical robot according to claim 1, wherein, the first arm further comprises a sensor for detecting current signals of the actuating device, the at least one processor is electrically connected to the sensor, and the at least one processor is further configured for:

receiving the current signals of the actuating device detected by the sensor;
determining whether the first arm receives the external force, and/or, determining the joint assembly of the first arm which receives the external force according to changes of the current signals; and
determining the first joint assembly based on the joint assembly which receives the external force.

9. The surgical robot according to claim 8, wherein, the sensor is configured for detecting the current signals at intervals of a first cycle, the step of determining whether the first arm receives the external force, and/or, determining the joint assembly of the first arm which receives the external force according to the changes of the current signals, comprises:

calculating a rate of change of detected adjacent current signals; and
in response to that the rate of change exceeds a first threshold, determining that the first arm receives the external force, and/or, determining the joint assembly which receives the external force according to the current signals of which the rate of change exceeds the first threshold.

10. The surgical robot according to claim 9, wherein, the sensor is configured for detecting the current signals at intervals of a second cycle, and the second cycle is larger than the first cycle, the step of determining whether the first arm receives the external force, and/or, determining the joint assembly of the first arm which receives the external force according to the changes of the current signals, comprises:

calculating the rate of change of the detected adjacent current signals; and
in response to that the rate of change exceeds a second threshold, determining that the first arm receives the external force, and/or, determining the joint assembly which receives the external force according to the current signals of which the rate of change exceeds the second threshold, wherein the second threshold is smaller than the first threshold.

11. The surgical robot according to claim 8, wherein, the step of determining whether the first arm receives the external force, and/or, determining the joint assembly of the first arm which receives the external force according to the changes of the current signals, comprises:
calculating a current difference between the current signals before the first arm receives the external force and the current signals when the first arm receives the external force; and determining whether the current difference exceeds a third threshold, in response to that the current difference exceeds the third threshold, determining that the first arm receives the external force, and/or, determining the joint assembly which receives the external force according to the current signals of which the current difference exceeds the third threshold.

12. The surgical robot according to claim 8, wherein, the step of determining the first joint assembly based on the joint assembly which receives the external force, comprises:

determining that the joint assembly comprises at least one of the joint assembly of the first arm that receives the external force, and the joint assembly in proximity to a proximal end of the joint assembly which receives the external force; or,
determining that the joint assembly comprises at least one of the joint assembly of the first arm that receives the external force, and the joint assembly in proximity to a proximal end of the joint assembly which receives the external force, and further comprises the joint assembly in proximity to a distal end of the joint assembly which receives the external force.

13. The surgical robot according to claim 2, wherein, when the second torque is a torque for braking the first joint assembly, the at least one processor is configured for:

obtaining an initial joint position and a current joint position of the first joint assembly detected by a sensor;
calculating a joint speed of the first joint assembly based on the initial joint position, the current joint position, and a time during which the initial joint position changes to the current joint position; and
determining the second torque outputted by the actuating device corresponding to the first joint assembly based on the joint speed.

14. The surgical robot according to claim 1, wherein, the surgical robot comprises a slave operating device and an operating device which is configured to control the slave operating device to move, the at least one arm comprises an arm of the slave operating device and an arm of the operating device, and the at least one arm comprises the first arm, the first arm comprises the arm of the operating device, and the step of adjusting, in response to the external force received by the first arm, the output torque of the actuating device corresponding to the first joint assembly of the joint assembly to balance the gravitational torque of the load of the distal end of the first joint assembly, and to restore the joint position of the first joint assembly or brake the first joint assembly, comprises:
when a doctor is in an unreadiness state, in response to the external force received by the first arm, adjusting the output torque of the actuating device corresponding to the first joint assembly of the joint assembly to balance the gravitational torque of the load of the distal end of the first joint assembly, and to restore the joint position of the first joint assembly or brake the first joint assembly, wherein the doctor being in the unreadiness state comprises at least one of following conditions:

it is detected that the doctor is not observing a display of a display device, it is detected that the doctor fails to activate the operating device, it is detected that the doctor is not seated in place, and it is detected that the doctor fails to be certified by a user authentication system.

15. The surgical robot according to claim 1, wherein, the surgical robot comprises a slave operating device and an operating device which controls the slave operating device to move, the at least one arm comprises an arm of the slave operating device and an arm of the operating device, and the at least one arm comprises the first arm, the first arm comprises the arm of the slave operating device, and the step of adjusting, in response to external force received by the first arm, the output torque of the actuating device corresponding to the first joint assembly of the joint assembly to balance the gravitational torque of the load of the distal end of the first joint assembly, and to restore the joint position of the first joint assembly or brake the first joint assembly, comprises:

in response to the first arm not being operated by the operating device, and/or, in response to the distal end of the first arm not mounted with a surgical instrument, and/or, in response to the distal end of the first arm mounted with the surgical instrument, and a type information and/or a state information of the distal end of an end effector that satisfies a safety condition, in response to the external force received by the first arm, adjusting the output torque of the actuating device corresponding to the first joint assembly of the joint assembly to balance the gravitational torque of the load of the distal end of the first joint assembly, and to restore the joint position of the first joint assembly or brake the first joint assembly.

16. The surgical robot according to claim 15, wherein, the first arm further comprises a sensor for detecting a joint position of the joint assembly, the at least one processor is electrically connected to the sensor, and the at least one processor is further configured for:

obtaining the joint position of the joint assembly of the first arm detected by the sensor;
calculating position parameters of a kinematic model of the first arm according to the joint position and a forward kinematics;
determining whether the position parameters of the kinematic model reach boundary parameters of an effective space of the first arm, wherein the effective space is at least a part of a Cartesian Space of the first arm; and
at least braking a second joint assembly in response to that the position parameters of the kinematic model reach the boundary parameters of the effective space, wherein the second joint assembly comprises a joint assembly of the first joint assembly which reaches the boundary parameters of the effective space.

17. The surgical robot according to claim 16, wherein, the at least one arm comprises a second arm, the second arm comprises another arm of the slave operating device, there is an overlapping part between the Cartesian Space of the first arm and a Cartesian Space of the second arm, in response to that position parameters of a kinematic model of the second arm is within the overlapping part, the effective space of the first arm is a part of the Cartesian Space of the first arm that excludes the overlapping part, and/or, in response to that the position parameters of the kinematic model of the second arm are not within the overlapping part, the effective space of the first arm is the whole Cartesian Space of the first arm.

18. The surgical robot according to claim 15, wherein, the at least one arm comprises a second arm, the second arm comprises another arm of the slave operating device, the second arm is operated by the operating device, the second arm further comprises a sensor for detecting current signals of an actuating device of the second arm, the at least one processor is electrically connected to the sensor, and the at least one processor is configured for:

recording a holding time in which the current signals reach a configured current threshold in response to that the current signals of the actuating device of the second arm detected by the sensor of the second arm reaches the configured current threshold;
determining whether the holding time reaches a time threshold; and
sending a command to the actuating device of the second arm to control the actuating device of the second arm to stop moving in response to that the holding time reaches the time threshold.

19. A control method for a surgical robot, **characterized in that**, surgical robot comprises at least one arm, the at least one arm comprises a joint assembly and an actuating device for actuating the joint assembly, the at least one arm comprises a first arm; and the control method comprises steps of:

adjusting, in response to an external force received by the first arm, an output torque of the actuating device corresponding to a first joint assembly of the joint assembly of the first arm to balance a gravitational torque of a load of a distal end of the first joint assembly, and to restore a joint position of the first joint assembly or brake the first joint assembly.

20. A computer-readable storage medium, **characterized by** being suitable for a surgical robot, wherein the surgical robot comprises at least one arm, the at least one arm comprises a joint assembly and an actuating device for actuating the joint assembly, and the computer-readable storage medium stores a computer program, which is configured for being loaded and executed by a processer to implement the steps of the control method according to claim 19.

FIG. 1

FIG. 2

FIG. 3

240'

FIG. 4

410    430    420

FIG. 5

start

no

determining whether
a collision happens        S1

yes

adjusting an output torque of an actuating device corresponding to
a first joint assembly of the joint assemblies of the first arm to
balance a gravitational torque of a load of a distal end of the first
joint assembly, and to restore a joint position of the first joint
assembly or braking the first joint assembly        S2

end

FIG. 6

| receiving current signals of an actuating device  detected by a sensor | S101 |

| determining whether the first arm and/or the device attached to the distal end of the first arm has a collision, and/or, determining the joint assembly where the crash point is located while the collision happening according to the changes of the current signals | S102 |

FIG. 7

| calculating a rate of change of the detected adjacent current signals | S1021 |

whether the rate of change exceeds a first threshold — S1022

no

yes

| determining that the first arm and/or the device attached to the distal end of the first arm has the collision, and/or, determining the joint assembly where the crash point is located while the collision happening according to the joint assembly corresponding to the current signals of which the rate of change exceeds the first threshold | S1023 |

FIG. 8

calculating a rate of change of the detected adjacent current signals S1021'

whether the rate of change exceeds a second threshold S1022'

no

yes

determining that the first arm and/or the device attached to the distal end of the first arm has the collision, and/or, determining the joint assembly where the crash point is located while the collision happening according to the joint assembly corresponding to the current signals of which the rate of change exceeds the second threshold S1023'

FIG. 9

calculating a current difference between the current signals and the current signals detected before the collision S1021"

whether the current difference exceeds a third threshold S1022"

no

yes

determining that the first arm and/or the device attached to the distal end of the first arm has the collision, and/or, determining the joint assembly where the crash point is located while the collision happening according to the joint assembly corresponding to the current signals of which the current difference exceeds the third threshold S1023"

FIG. 10

receiving a force signal of the joint assembly detected by a sensor — S111

determining whether the first arm and/or the device attached to the distal end of the first arm has the collision, and/or, determining the joint assembly where the crash point is located while the collision happening according to a change of the force signal — S112

## FIG. 11

determining whether the position parameters of the kinematic model of the first arm and/or the device attached to the distal end of the first arm are the same with the position parameters of the kinematic model of the second arm and/or the device attached to the second arm — S121

determining whether the first arm and/or the device attached to the distal end of the first arm has a collision and/or determining the joint assembly where the crash point is located while the collision happening according to the condition whether the position parameters are the same — S122

## FIG. 12

obtaining joint positions of at least the first joint assembly and a joint assembly which is located at the distal end of the first joint assembly which are detected by the sensors — S201

determining a desired output first torque of the actuating device corresponding to the first joint assembly by combination with the joint positions and a dynamics model associated with the first joint assembly — S202

## FIG. 13

obtaining linkage parameters of the first joint assembly and the joint assembly located at the distal end of the first joint assembly, building a coordinate system of linkage according to the linkage parameters    S2021

constructing the first dynamics model corresponding to the first joint assembly according to the coordinate system of linkage    S2022

determining unknown dynamics parameters of the first dynamics model    S2023

obtaining a second dynamics model by bringing the dynamics parameters into the first dynamics model    S2024

FIG. 14

obtaining an initial joint position and a current joint position of the first joint assembly detected by a sensor    S211

calculating a joint position variable of the first joint assembly based on the initial joint position and the current joint position    S212

determining the second torque outputted by the actuating device corresponding to the first joint assembly based on the joint position variable    S213

FIG. 15

| obtaining the current joint position of the first joint assembly detected by the sensor | S221 |

| determining whether the current joint position reaches a first limiting joint position of the first joint assembly | S222 |

| calculating a joint position variable between the current joint position and the first limiting joint position | S223 |

| determining the second torque outputted by the actuating device corresponding to the first joint assembly based on the joint position variable | S224 |

FIG. 16

| obtaining an initial joint position and a current joint position of the first joint assembly detected by a sensor | S231 |

| calculating a joint speed of the first joint assembly based on the initial joint position, the current joint position, and a time during which the initial joint position changes to the current joint position | S232 |

| determining the second torque outputted by the actuating device corresponding to the first joint assembly based on the joint speed | S233 |

FIG. 17

| recording a holding time of the first joint assemblies at the current joint position during a duration of the collision | S31 |

| determining whether the holding time reaches a configured time threshold | S32 |

| updating the current joint position to be the initial joint position | S33 |

FIG. 18

| obtaining the joint position of the joint assembly of the first arm detected by the sensor | S41 |

| calculating position parameters of a kinematic model of the first arm according to the joint position and a forward kinematics | S42 |

| determining whether the position parameters of the kinematic model reach boundary parameters of an effective space of the first arm | S43 |

| at least braking a second joint assembly | S44 |

FIG. 19

start

no

determining whether the condition is satisfied    S10

no

yes

adjusting the output torque of the actuating device corresponding to the first joint assembly of the joint assemblies to balance the gravitational torque of the load of the distal end of the corresponding first joint assembly    S11

determining whether a collision happens    S1

yes

adjusting an output torque of an actuating device corresponding to a first joint assembly of the joint assemblies of the first arm for balancing a gravitational torque generated by a load of a distal end of the first joint assembly, and restoring a joint position of the first joint assembly or braking the first joint assembly    S2

end

FIG. 20

Control apparatus

Memory
503

Program
505

Processor
501

Communication bus 504

Communication
interface
502

FIG. 21

## INTERNATIONAL SEARCH REPORT

| | International application No. |
|---|---|
| | **PCT/CN2022/085047** |

**A. CLASSIFICATION OF SUBJECT MATTER**

A61B 34/30(2016.01)i

According to International Patent Classification (IPC) or to both national classification and IPC

**B. FIELDS SEARCHED**

Minimum documentation searched (classification system followed by classification symbols)

A61B34/-

Documentation searched other than minimum documentation to the extent that such documents are included in the fields searched

Electronic data base consulted during the international search (name of data base and, where practicable, search terms used)

CNABS, CNTXT, WPI, EPODOC, CNKI: 精锋医疗, 机器人, 手术, 臂, 碰撞, 外力, 缓冲, 扭矩, 力矩, 补偿, 关节, robot, operation, surgery, arm, crash, collision, external force, buffer, torque, moment, compensation, joint

**C. DOCUMENTS CONSIDERED TO BE RELEVANT**

| Category* | Citation of document, with indication, where appropriate, of the relevant passages | Relevant to claim No. |
|---|---|---|
| X | CN 112566583 A (COVIDIEN LP) 26 March 2021 (2021-03-26)<br>description, pages [0023]-[0077], and figures 1-8 | 1-20 |
| X | US 2020282558 A1 (LINKDYN ROBOTICS INC.) 10 September 2020 (2020-09-10)<br>description, paragraphs [0079]-[0080], and figures 1-13 | 1-20 |
| X | US 2019084154 A1 (HIWIN TECHNOLOGIES CORP.) 21 March 2019 (2019-03-21)<br>description, paragraphs [0050]-[0077], and figures 1-8 | 1-20 |
| A | CN 112384336 A (CMR SURGICAL LIMITED) 19 February 2021 (2021-02-19)<br>entire document | 1-20 |
| A | CN 114126528 A (VERB SURGICAL INC.) 01 March 2022 (2022-03-01)<br>entire document | 1-20 |
| A | CN 111673733 A (SOUTH CHINA UNIVERSITY OF TECHNOLOGY) 18 September 2020<br>(2020-09-18)<br>entire document | 1-20 |

☐ Further documents are listed in the continuation of Box C. ☑ See patent family annex.

| * Special categories of cited documents: | "T" | later document published after the international filing date or priority date and not in conflict with the application but cited to understand the principle or theory underlying the invention |
|---|---|---|
| "A" document defining the general state of the art which is not considered to be of particular relevance | | |
| "E" earlier application or patent but published on or after the international filing date | "X" | document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" document referring to an oral disclosure, use, exhibition or other means | | |
| "P" document published prior to the international filing date but later than the priority date claimed | "&" | document member of the same patent family |

| Date of the actual completion of the international search | Date of mailing of the international search report |
|---|---|
| **07 June 2022** | **01 July 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2022/085047**

| Patent document cited in search report | | | Publication date (day/month/year) | Patent family member(s) | | | Publication date (day/month/year) |
|---|---|---|---|---|---|---|---|
| CN | 112566583 | A | 26 March 2021 | WO | 2020246997 | A1 | 10 December 2020 |
| | | | | EP | 3975907 | A1 | 06 April 2022 |
| US | 2020282558 | A1 | 10 September 2020 | | None | | |
| US | 2019084154 | A1 | 21 March 2019 | JP | 2019193965 | A | 07 November 2019 |
| | | | | DE | 102018209594 | A1 | 21 March 2019 |
| | | | | TW | I642523 | B | 01 December 2018 |
| CN | 112384336 | A | 19 February 2021 | US | 2021267700 | A1 | 02 September 2021 |
| | | | | WO | 2020002922 | A1 | 02 January 2020 |
| | | | | BR | 112020026857 | A2 | 06 April 2021 |
| | | | | GB | 201810754 | D0 | 15 August 2018 |
| | | | | JP | 2021531846 | A | 25 November 2021 |
| | | | | EP | 3814071 | A1 | 05 May 2021 |
| | | | | AU | 2019295378 | A1 | 11 February 2021 |
| CN | 114126528 | A | 01 March 2022 | | None | | |
| CN | 111673733 | A | 18 September 2020 | | None | | |

Form PCT/ISA/210 (patent family annex) (January 2015)

**REFERENCES CITED IN THE DESCRIPTION**

**Patent documents cited in the description**

- CN 202110628500X